# EUROPEAN PATENT SPECIFICATION

(11) **EP 3 792 259 B1**
(45) Date of publication and mention of the grant of the patent: **29.11.2023**
(21) Application number: 20187479.9
(22) Date of filing: 23.07.2020
(51) Int. Cl.: C07D 413/14, C07D 417/14, H10K 85/60

(54) **HETEROCYCLIC COMPOUND AND ORGANIC ELECTROLUMINESCENT DEVICE COMPRISING THE SAME**
HETEROCYCLISCHE VERBINDUNG UND ORGANISCHE ELEKTROLUMINESZENTE VORRICHTUNG DAMIT
COMPOSÉ HÉTÉROCYCLIQUE ET DISPOSITIF ÉLECTROLUMINESCENT ORGANIQUE LE COMPRENANT

(30) Priority: 16.09.2019 CN 201910872691
(43) Date of publication of application: 17.03.2021
(73) Proprietor: Changchun Hyperions Technology Co., Ltd., Bayhood Science and Technology Development Zone Changchun City Jilin 130000 (CN)
(72) Inventor: DU, Mingzhu, Changchun City, Jilin 130000 (CN); LIU, Hui, Changchun City, Jilin 130000 (CN); ZHAO, Qian, Changchun City, Jilin 130000 (CN); WANG, Xiaohui, Changchun City, Jilin 130000 (CN)
(74) Representative: Hahner, Ralph

(56) References cited:
- EP-A1- 3 432 688
- CN-A- 109 761 967
- CN-A- 111 004 226
- US-A1- 2008 091 025

## Description

### TECHNICAL FIELD

The present disclosure relates to the field of organic electroluminescent materials and specifically, relates to a heterocyclic compound and an organic electroluminescent device comprising the same.

### BACKGROUND

An organic light-emitting diode (OLED) is an all-solid-state light emitting device, and has the advantages of high brightness, high contrast, high definition, wide angle of view, wide color gamut, ultra-thin thickness, ultra-light weight, low power consumption, wide temperature range, self-luminescence, high luminescence efficiency, short response time, transparency, flexibility, and the like. The OLED has been commercially used in the fields of mobile phone, television, micro display and the like, has been called dreamy display in the industry, and will become the most promising new display technology in the future.

Since total reflection will occur at the interface between the Indium tin oxide (ITO) film and glass substrate of the OLED or at the interface of the glass substrate and air, only 20% of the emitted light can be utilized, and the remaining 80% of the light is limited to being inside the device and converted to heat, which adversely affects the device. In the top-emitter, a cover layer with high refractive index is usually provided on the outside of the translucent electrode to adjust the optical interference distance, reduce the total reflection effect of the device, and improve the light extraction efficiency. The Japanese Patent Publication No. JP2017123341 discloses a cover layer material of aromatic amine derivatives, which improves the light extraction efficiency to some extent, but the refractive index of this material is only about 1.90. In addition, the types of cover layer materials are relatively single, so it is of great application significance to develop new cover layer materials. Furthermore, CN 109761967 A discloses a series of compounds based on heteroaryl amine structures and application thereof in OLEDs.

### SUMMARY

The present disclosure provides a heterocyclic compound and an organic electroluminescent device comprising the same.

The present disclosure provides a heterocyclic compound having a structure represented by Formula (I):

A-L-B Formula (I)

wherein L is selected from any one of the following groups: 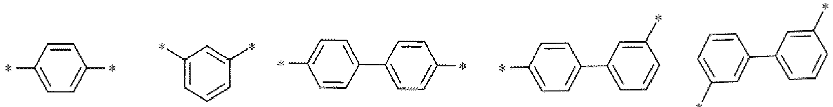 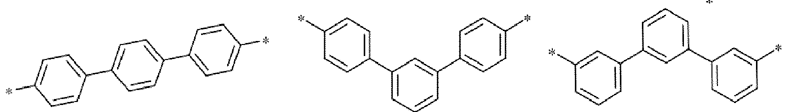 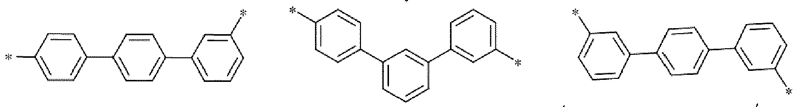 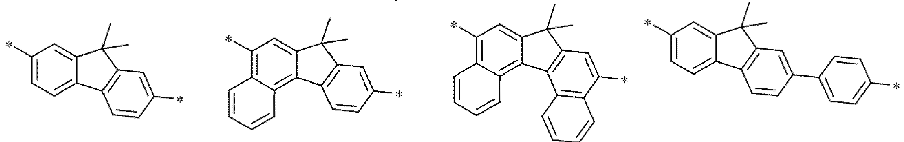 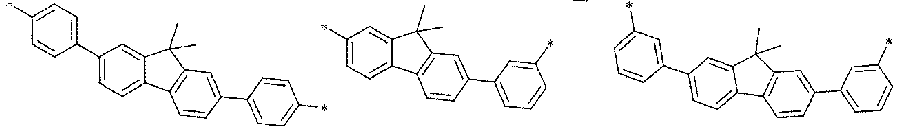 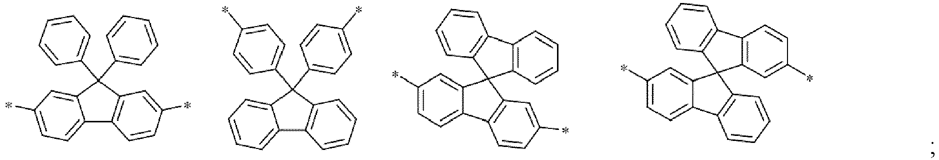 and
A and B are independently selected from any one of Formula (II) or Formula (III): 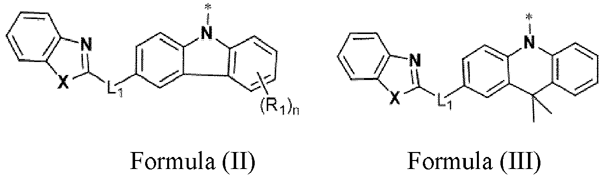
wherein X is oxygen (O) or sulphur (S);
L₁ is a single bond, phenyl or biphenyl;
n is 0, 1, 2, 3 or 4; and
R₁ is hydrogen (H), C1 - C15 alkyl or C6 - C30 aryl, or two adjacent R₁ groups are bonded to form a ring structure.

The present disclosure further provides an organic electroluminescent device comprising the heterocyclic compound.

The present disclosure has the following beneficial effects:
Compared with the existing art, the heterocyclic compound provided by the present disclosure has a high refractive index which can reach 1.95 to 2.10, such that when used as a cover layer material, it can improve the transmittance of a semi-transmissive electrode, adjust the light extraction direction and improve the light extraction efficiency. Moreover, the heterocyclic compound provided by the present disclosure improves the glass transition temperature through both structure optimization and introduction of a five-membered heterocyclic ring containing nitrogen or a six-membered heterocyclic ring containing nitrogen, benzoxazole or a benzothiazole group, to allow better film-forming property and stability, such that when as used as the cover layer material of an organic electroluminescent device, it can effectively increase the service life of the device.

The organic electroluminescent device containing the heterocyclic compound further provided by the present disclosure has high luminescence efficiency and great lifetime performance.

### BRIEF DESCRIPTION OF DRAWINGS

FIG. 1 shows a ¹H nuclear magnetic resonance (¹H NMR) diagram of Compound 9 prepared in Example 1 of the present disclosure;
FIG. 2 shows a ¹H NMR diagram of Compound 18 prepared in Example 4 of the present disclosure;
FIG. 3 shows a ¹H NMR diagram of Compound 34 prepared in Example 7 of the present disclosure;
FIG. 4 shows a ¹H NMR diagram of Compound 51 (not according to the present invention) prepared in Example 8 of the present disclosure;
FIG. 5 shows a ¹H NMR diagram of Compound 95 prepared in Example 9 of the present disclosure; and
FIG. 6 shows a ¹H NMR diagram of Compound 111 prepared in Example 14 of the present disclosure.

### DETAILED DESCRIPTION

The present disclosure will be described clearly and completely in conjunction with technical solutions in the specific examples of the present disclosure. Apparently, the examples described are part, not all, of the examples of the present disclosure. Based on the examples of the present disclosure, all other examples obtained by those skilled in the art are within the scope of the present disclosure on the premise that no creative work is done.

The present disclosure first provides a heterocyclic compound having a structure represented by Formula (I):

A-L-B Formula (I)

wherein L is selected from any one of the following groups: 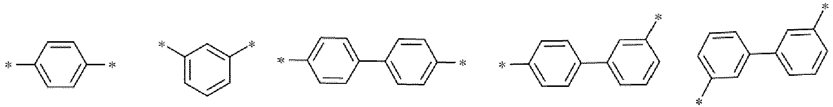 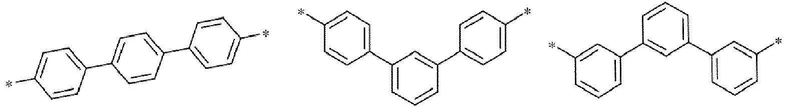 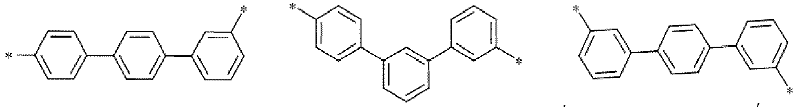 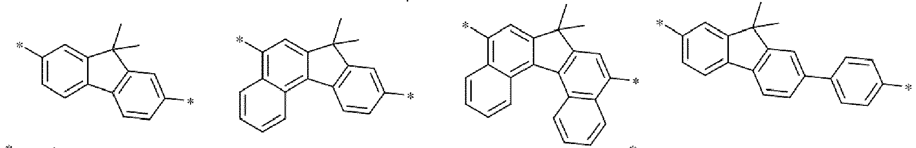 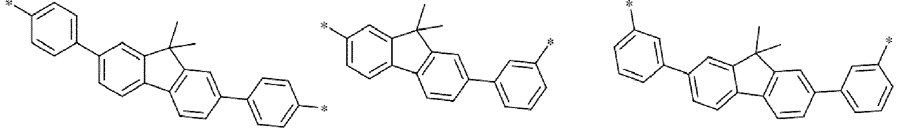 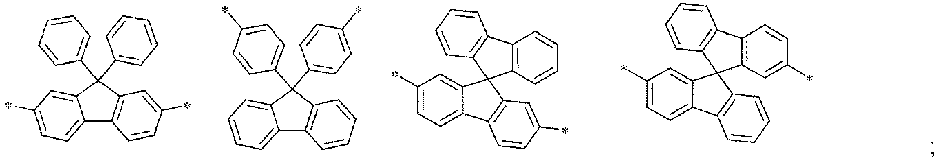 and
A and B are independently selected from any one of Formula (II) or Formula (III): 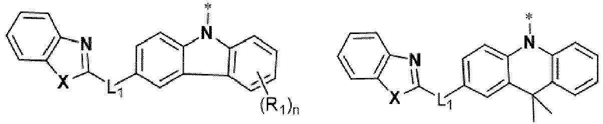 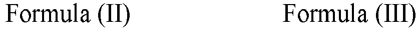
wherein X is O or S;
L₁ is a single bond, phenyl or biphenyl;
n is 0, 1, 2, 3 or 4; and
R₁ is H, C1 - C15 alkyl or C6 - C30 aryl, or two adjacent R₁ groups are bonded to form a ring structure.

In referring "substituted or unsubstituted" as described in the present disclosure, a substituent is independently selected from a deuterium atom, cyano, nitro, a halogen atom, C1 - C10 alkyl, C1 - C10 alkoxy, C1 - C10 alkylthio, C6 - C30 aryl, C6 - C30 aryloxy, C6 - C30 arylthio, C3 - C30 heteroaryl, C1 - C30 silylalkyl, C2 - C10 alkylamino, C6 - C30 arylamino, or a combination thereof, for example, a deuterium atom, cyano, nitro, halogen, methyl, ethyl, propyl, isopropyl, t-butyl, methoxy, methylthio, phenyl, biphenyl, triphenyl, naphthyl, anthryl, phenanthryl, benzophenanthryl, perylene, pyrenyl, fluorenyl, 9,9-dimethylfluorenyl, benzyl, phenoxy, thiophenyl, diphenylamino, dimethylamido, carbazolyl, 9-phenylcarbazolyl, furyl, thienyl, triphenylsilyl, trimethylsilyl, trifluoromethyl, phenothiazinyl, phenoxazine, acridinyl, pyridyl, pyrazinyl, triazinyl, pyrimidyl, and the like, but not limited thereto. The substituent may be any substituent other than those enumerated above as long as the technical effect of the present disclosure can be achieved. There may be one or more substituents, and when there is more than one substituent, each of the multiple substituents is the same or different.

The "alkyl" described in the present disclosure refers to a hydrocarbon radical obtained by removing one hydrogen atom from an alkane molecule, and it may be linear alkyl, branched alkyl or cycloalkyl, and preferably has 1 to 15 carbon atoms, more preferably 1 to 10 carbon atoms, and particularly preferably 1 to 4 carbon atoms. Examples may include, but are not limited to, methyl, ethyl, propyl, isopropyl, normal-butyl, isobutyl, secondary-butyl, tertiary-butyl, pentyl, isopentyl, cyclopentyl, cyclohexyl, and the like.

The "alkoxy" described in the present disclosure refers to a group obtained after alkyl is bonded to an oxygen atom, that is, an "alkyl-O-" group, wherein the alkyl is defined as above. Examples may include, but are not limited to, methoxy, ethoxy, 2-propoxy, 2-cyclohexoxy, and the like.

The "alkylthio" described in the present disclosure refers to a group obtained after alkyl is bonded to a sulphur atom, that is, an "alkyl-S-" group, wherein the alkyl is defined as above.

The "aryl" described in the present disclosure refers to a general term of a monovalent group left after one hydrogen atom is removed from aromatic nucleus carbon of an aromatic hydrocarbon molecule, and it may be monocyclic aryl, polycyclic aryl or fused ring aryl, and preferably has 6 to 30 carbon atoms, more preferably 6 to 18 carbon atoms, and particularly preferably 6 to 14 carbon atoms. Examples may include, but are not limited to, phenyl, biphenyl, naphthyl, anthryl, phenanthryl, benzophenanthryl, perylene, pyrenyl, fluorenyl, benzofluorenyl, dibenzofluorenyl, spirodifluorenyl, and the like.

The "aryloxy" described in the present disclosure refers to a group obtained after aryl is bonded to an oxygen atom, that is, an "aryl-O-" group, wherein the aryl is defined as above.

The "arylthio" described in the present disclosure refers to a group obtained after aryl is bonded to a sulphur atom, that is, an "aryl-S-" group, wherein the aryl is defined as above.

The "heteroaryl" described in the present disclosure refers to a general term of a group obtained after one or more aromatic nucleus carbons in aryl are replaced with heteroatoms, wherein the heteroatom includes, but is not limited to, an oxygen atom, a sulfur atom, a nitrogen atom or a silicon atom, and the heteroaryl preferably has 1 to 25 carbon atoms, more preferably 2 to 20 carbon atoms, and particularly preferably 3 to 15 carbon atoms. The heteroaryl may be monocyclic, polycyclic or fused. Examples may include, but are not limited to, furyl, thienyl, pyridyl, pyrazinyl, pyrimidyl, phenothiazinyl, phenoxazinyl, benzopyrimidinyl, carbazolyl, triazinyl, benzothiazole, benzimidazolyl, dibenzothiophene, dibenzofuran, acridinyl, and the like. Examples of the silylalkyl described in the present disclosure include, but are not limited to, trimethylsilane, triethylsilane, triphenylsilane, trimethoxysilane, dimethoxyphenylsilane, diphenylmethylsilane, silane, diphenylenesilane, methylcyclobutylsilane, dimethylfuranosilane, and the like.

The "alkylamino" described in the present disclosure refers to a general term of a group obtained after a hydrogen atom in amino, *i.e*., -NH₂, is substituted with alkyl. Examples may include, but are not limited to, -N(CH₃)₂, -N(CH₂CH₃)₂, and the like.

The "arylamino" described in the present disclosure refers to a general term of a group obtained after a hydrogen atom in amino, *i.e*., -NH₂, is substituted with an aromatic group. The arylamino group may be further substituted with the substituent described in the present disclosure. Examples may include, but are not limited to, the following structures:

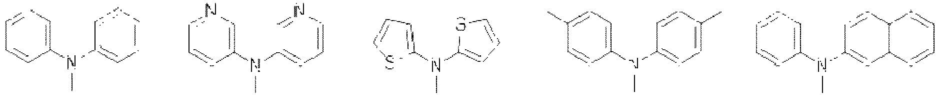

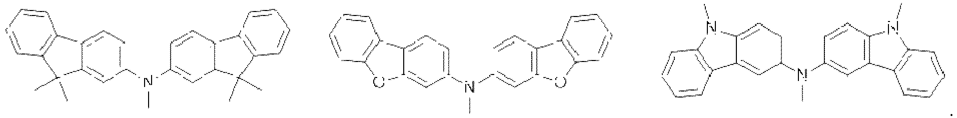

The expression "being bonded to form a ring structure" described in the present disclosure refers to that two groups are joined to each other through a chemical bond, which can be exemplified by the following formula:

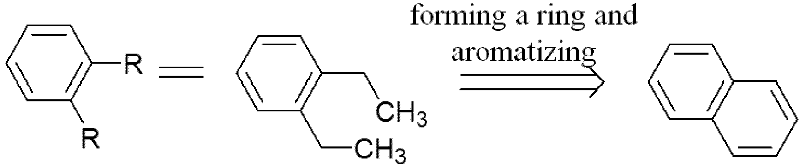

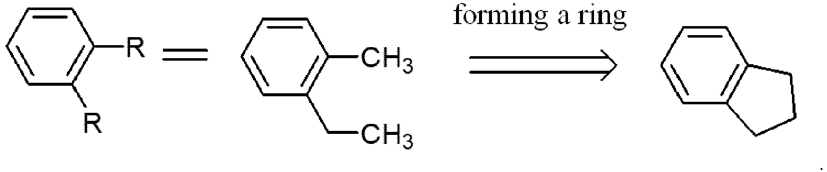

In the present disclosure, the ring formed through bonding may be a five-membered ring, a six-membered ring or a fused ring, for example, phenyl, naphthyl, cyclopentenyl, cyclohexanophenyl, quinolyl, isoquinolyl, dibenzothienyl, phenanthryl or pyrenyl, but not limited thereto.

L is selected from any one of the following groups:

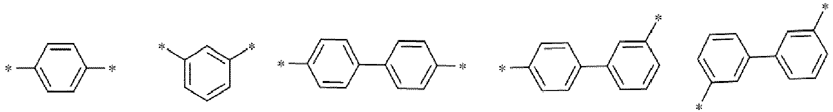

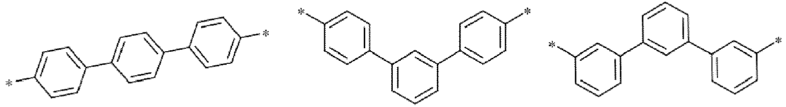

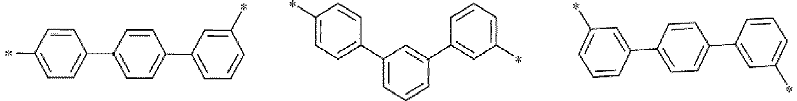

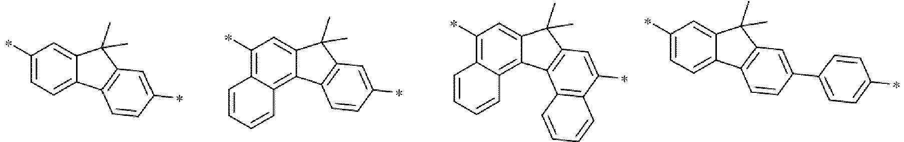

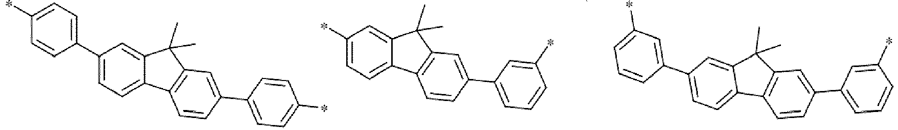

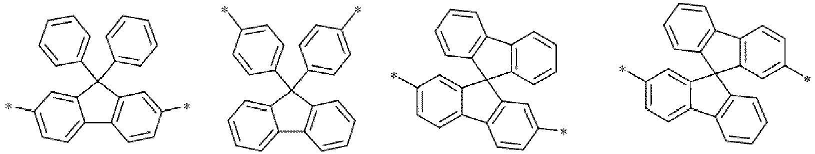

Preferably, R₁ is H, C1 - C4 alkyl, phenyl or biphenyl, or two adjacent R₁ groups are bonded to form a ring structure.

Preferably, A and B are independently selected from any one of the following formulas:

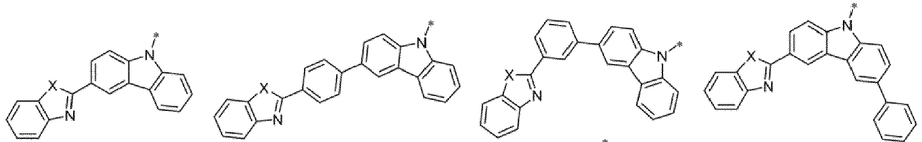

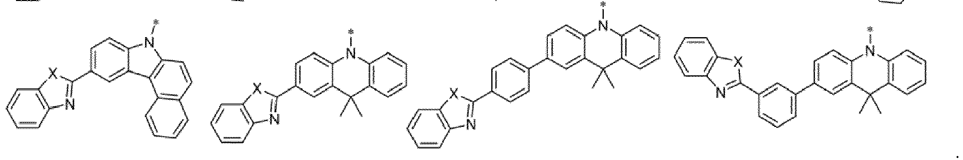

Preferably, the heterocyclic compound is selected from any one of the following compounds (compounds 42-88 and 127-190 are not according to the invention):

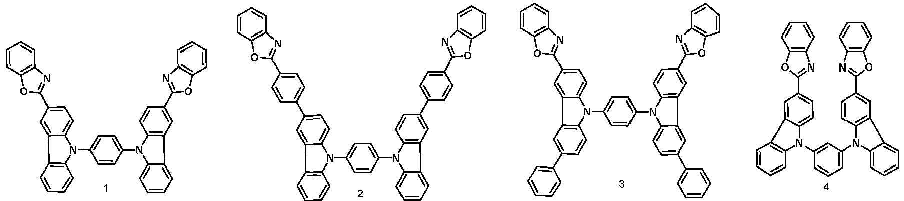

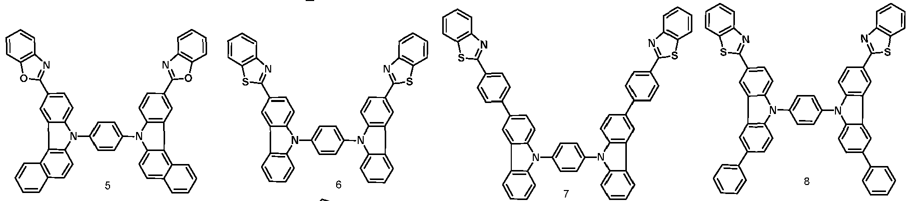

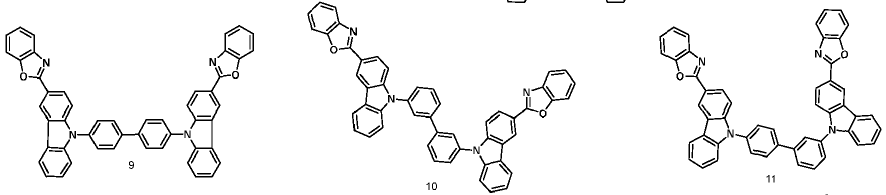

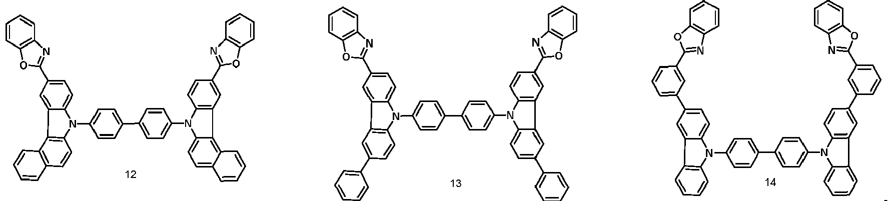

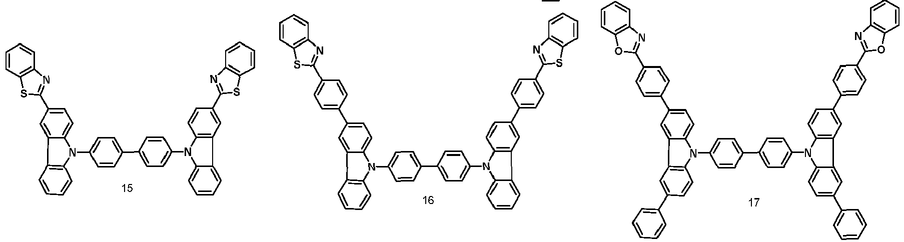

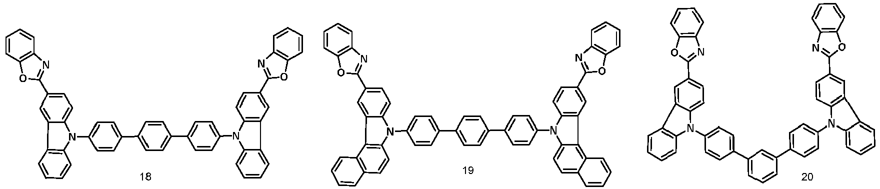

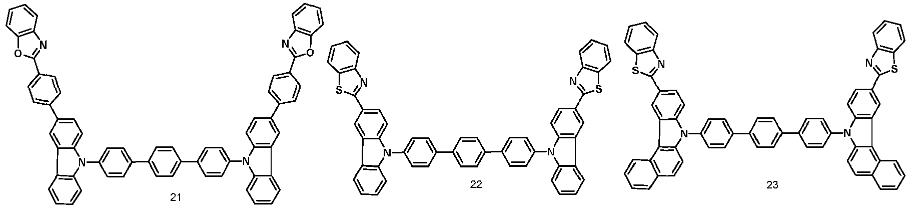

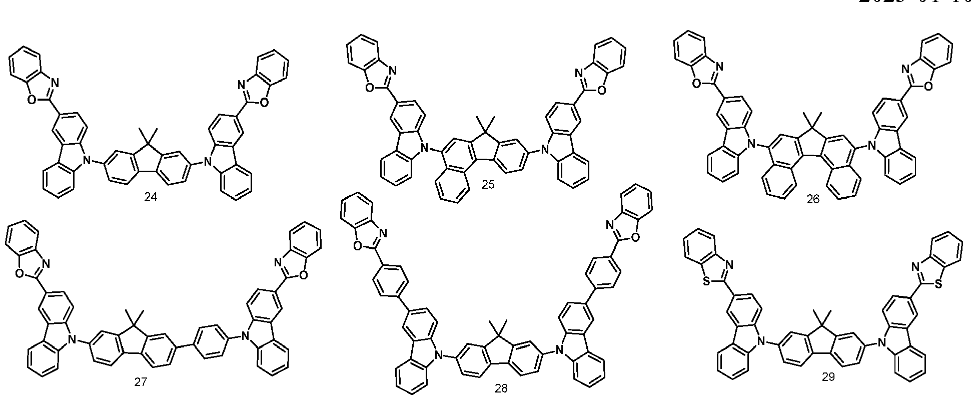

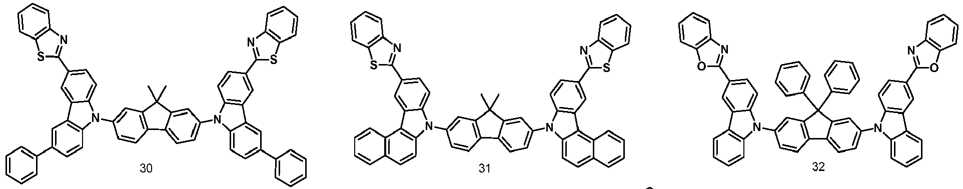

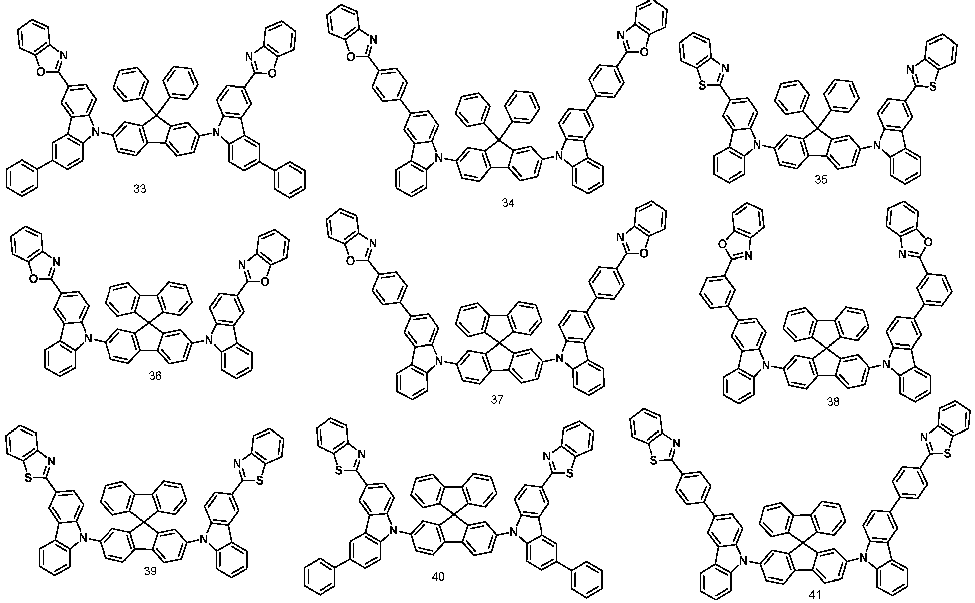

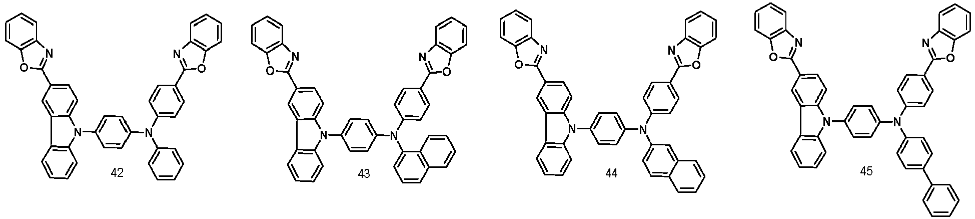

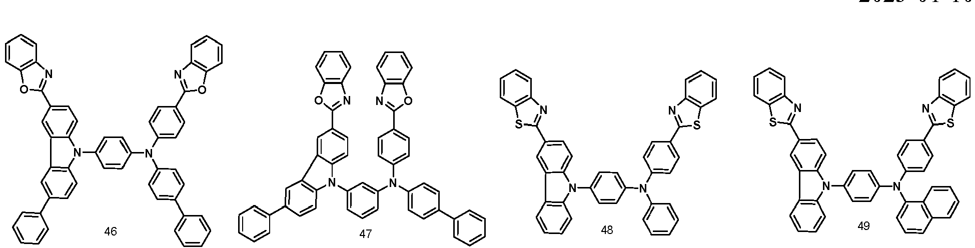

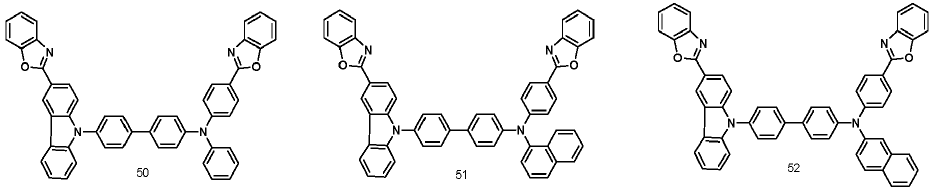

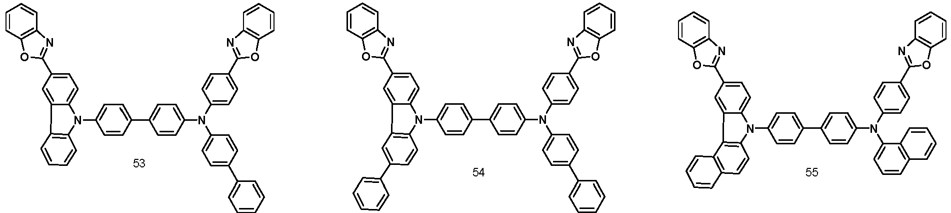

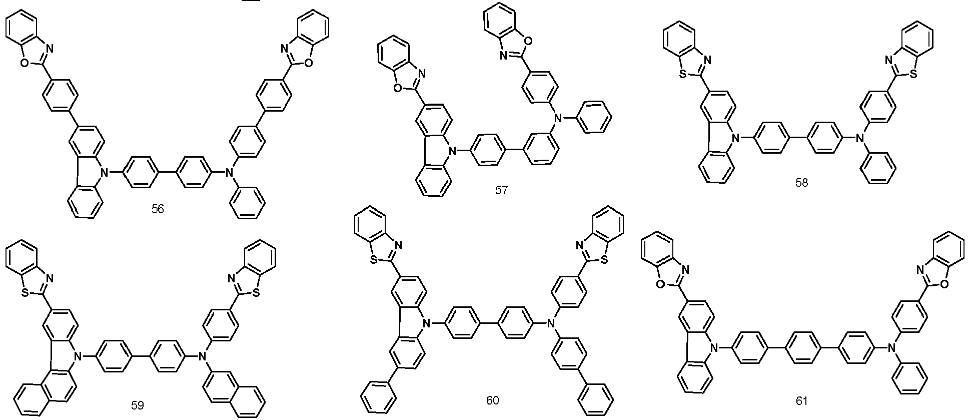

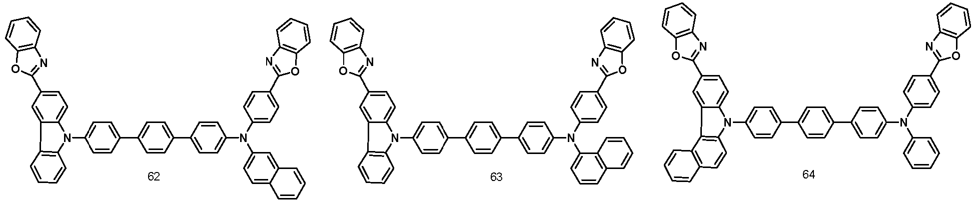

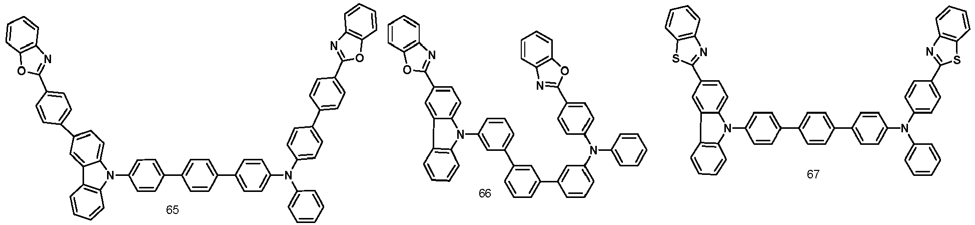

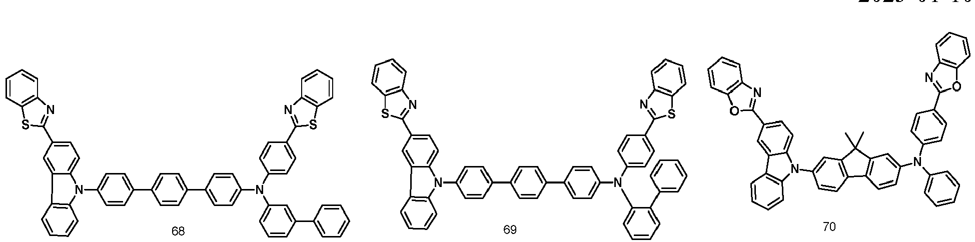

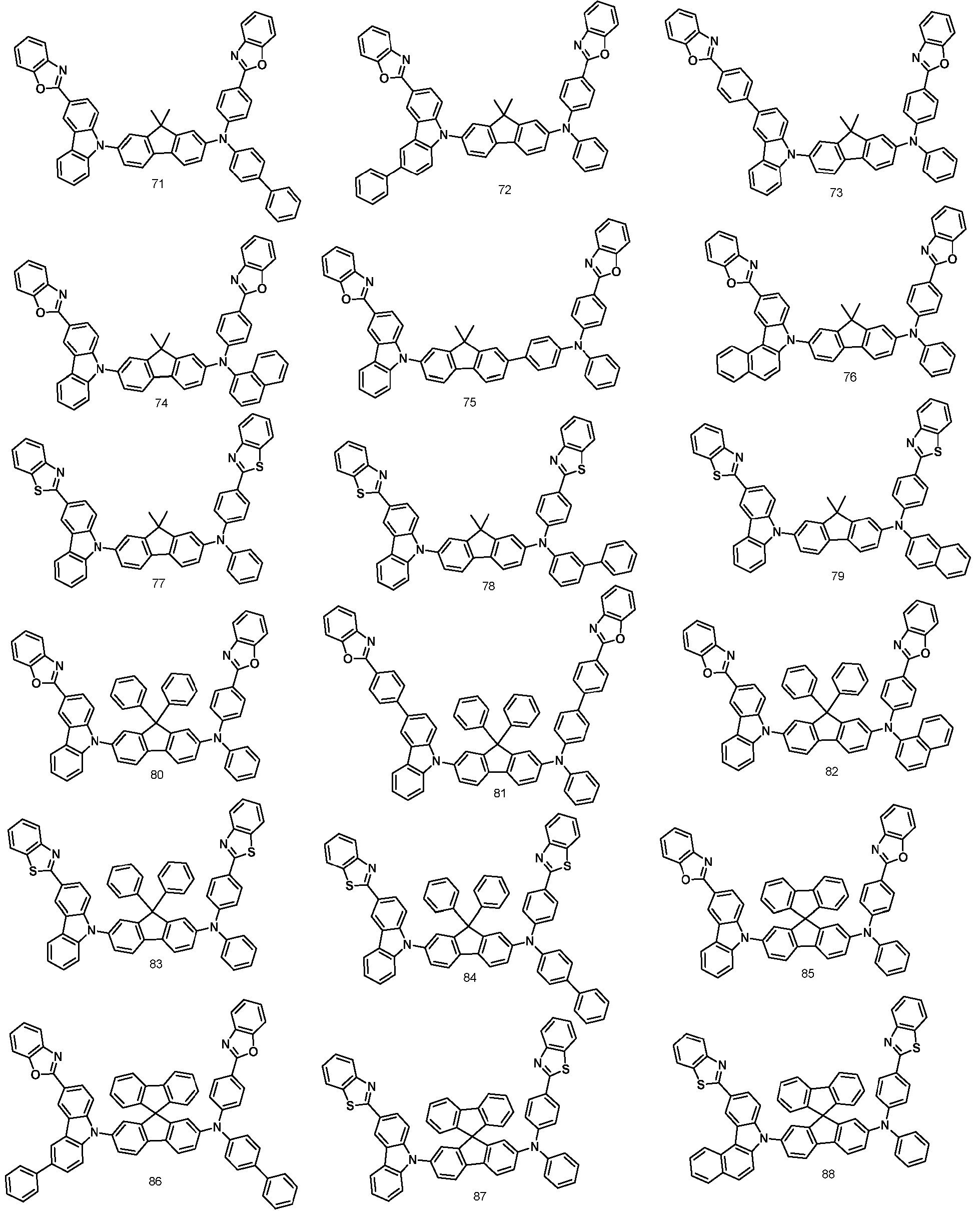

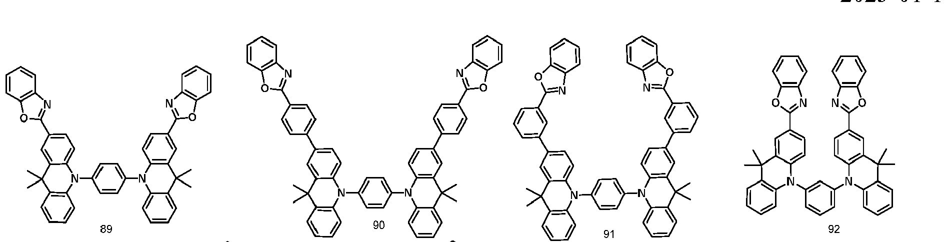

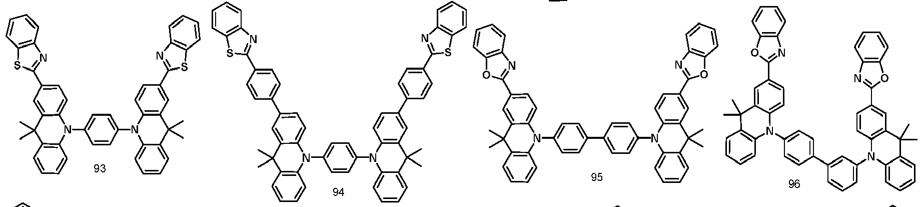

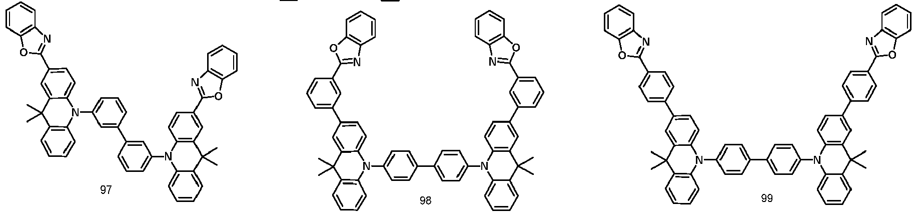

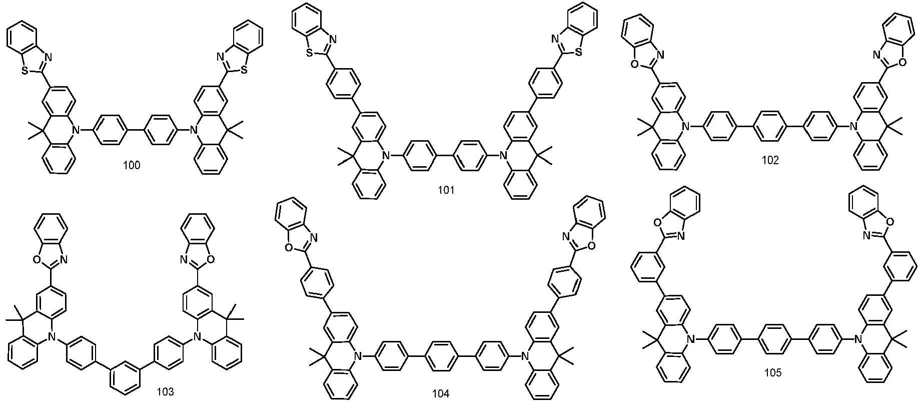

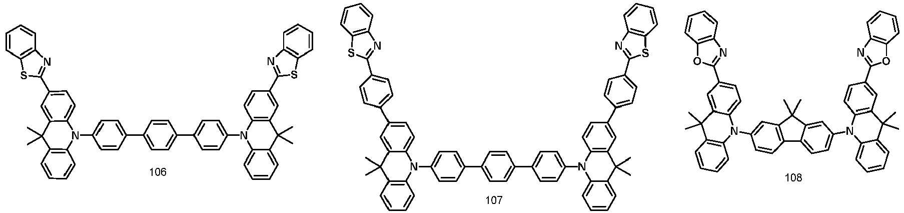

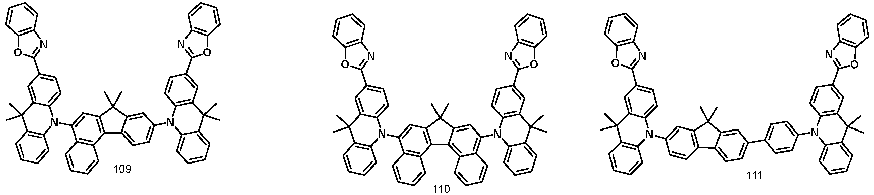

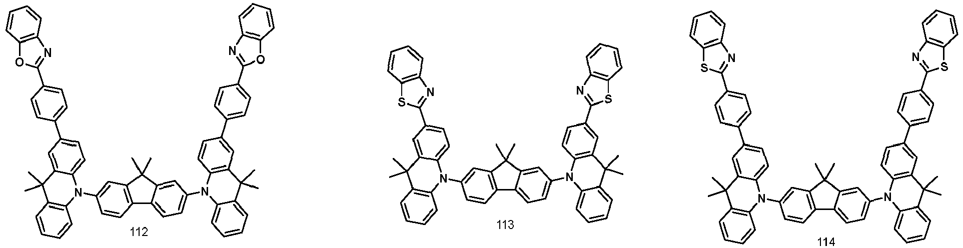

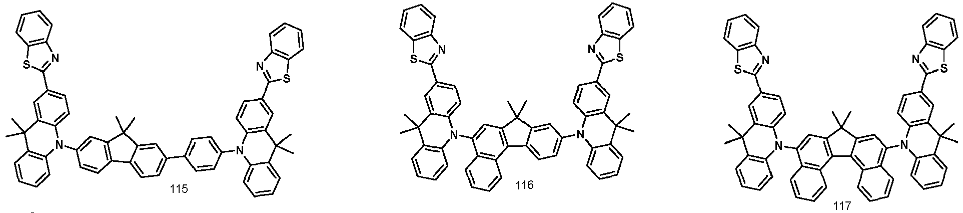

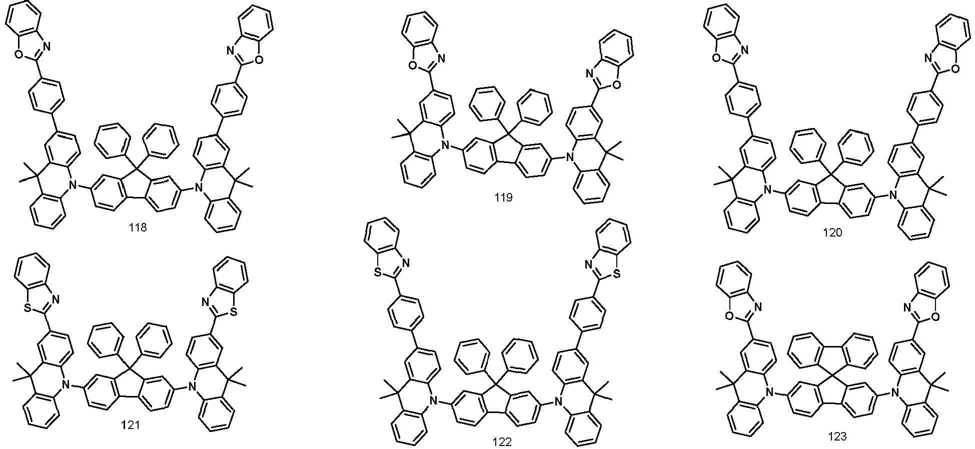

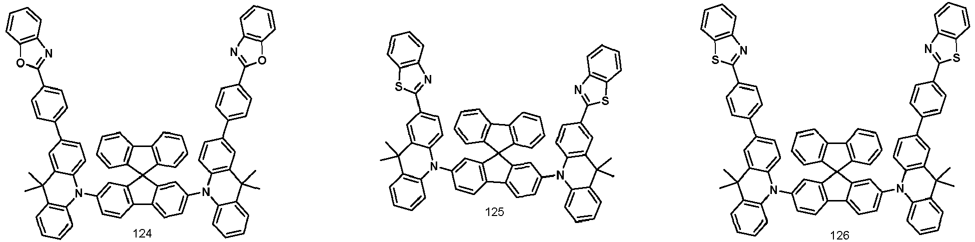

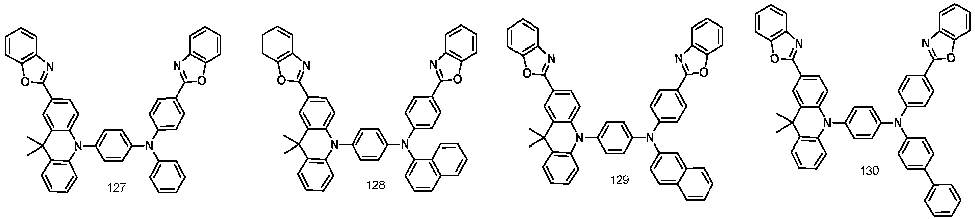

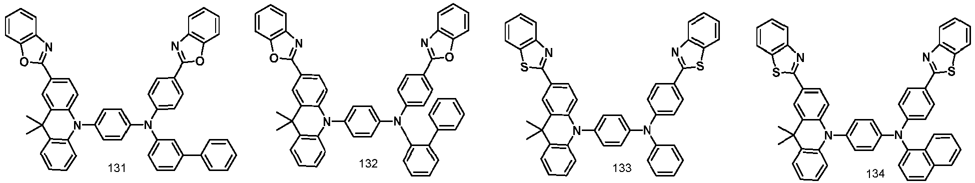

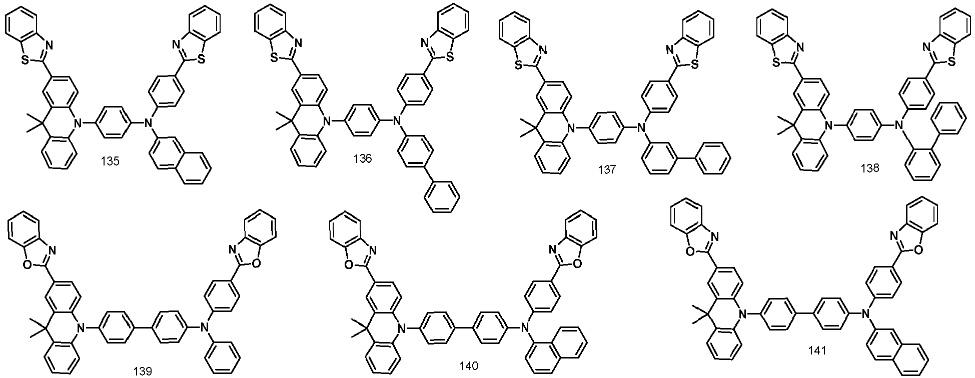

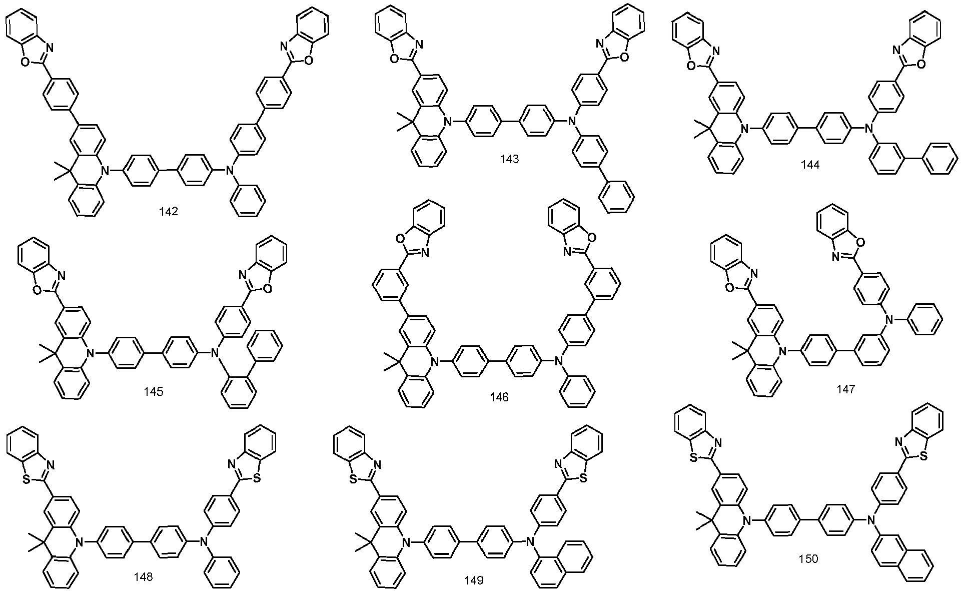

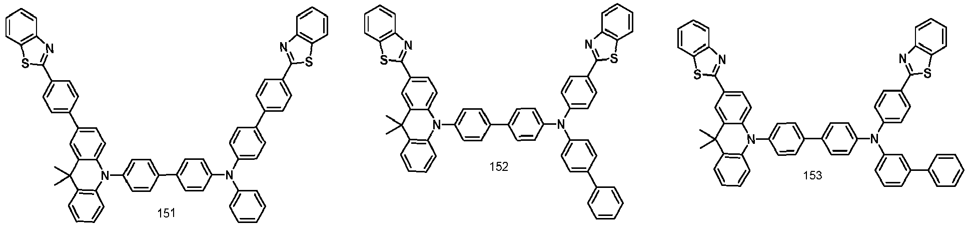

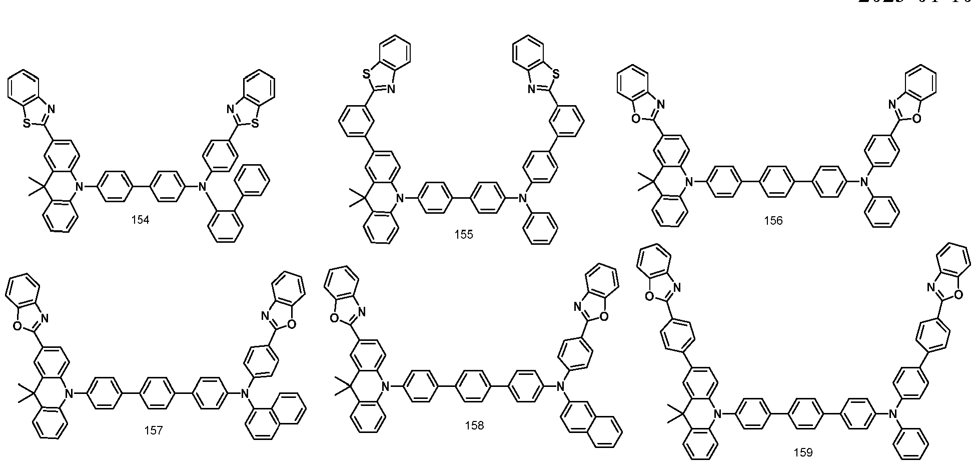

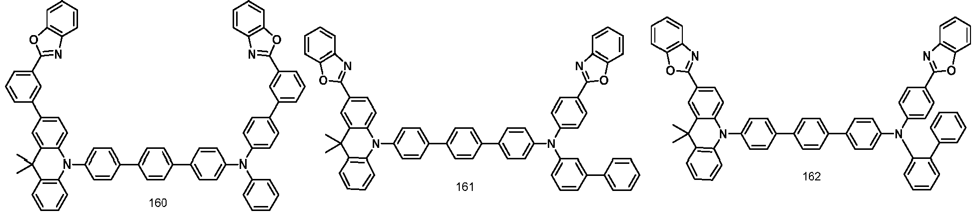

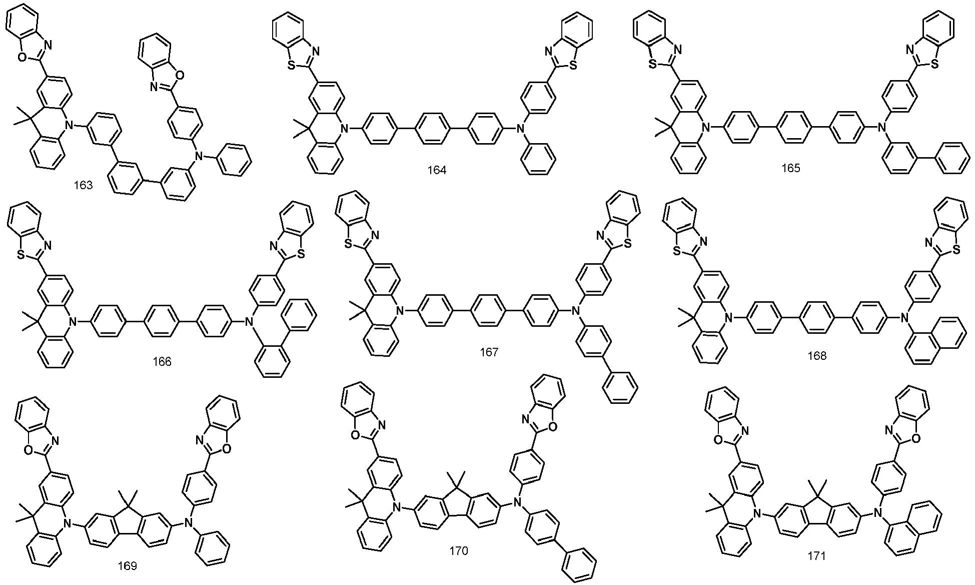

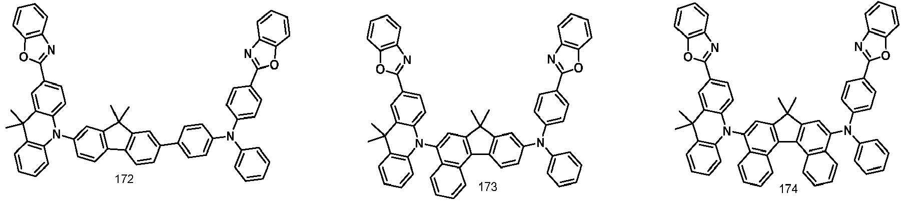

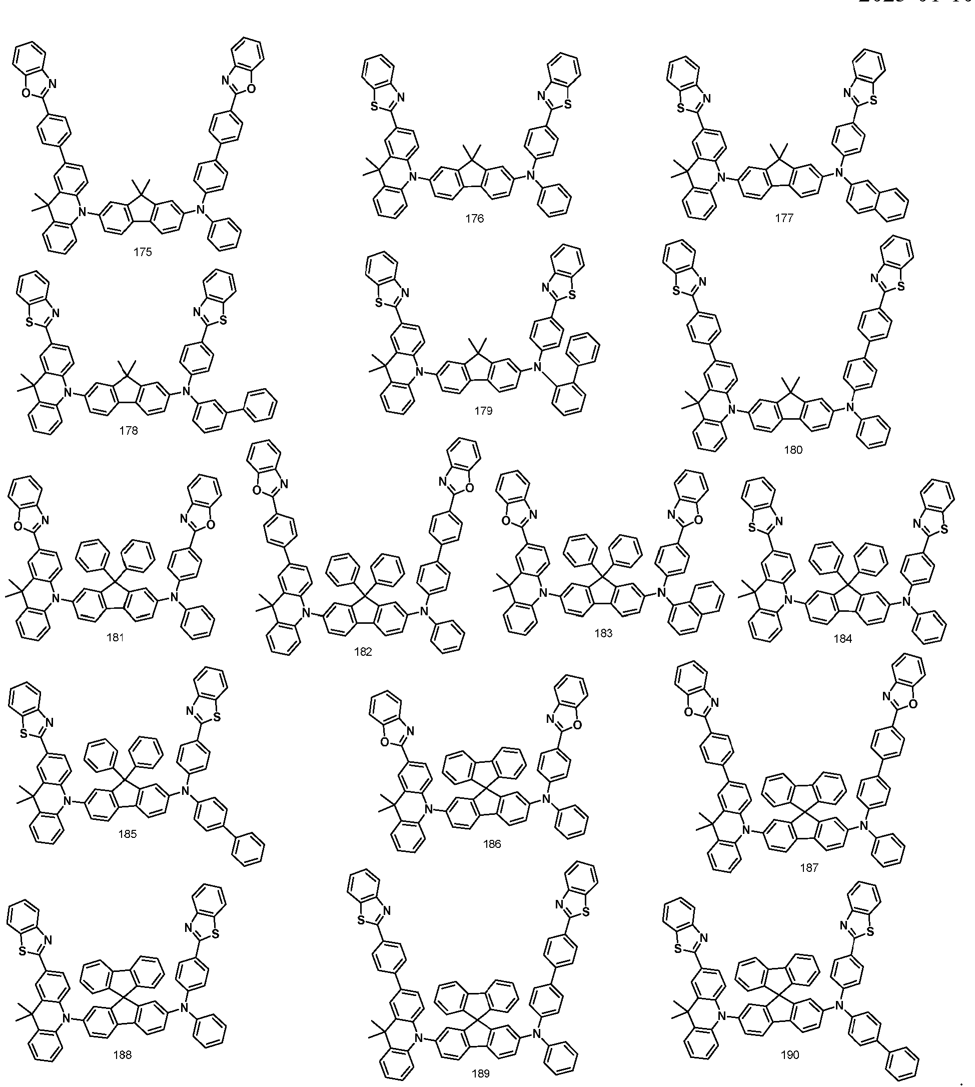

Some specific structure forms of the heterocyclic compound according to the present disclosure are illustrated above, but the present disclosure is not limited to these chemical structures. Any compound having a basic structure as shown in Formula (I) and having substituents as defined above shall be included.

The heterocyclic compound according to the present disclosure may be prepared through conventional coupled reaction. For example, the heterocyclic compound may be prepared according to the following synthetic route, but the present disclosure is not limited thereto.

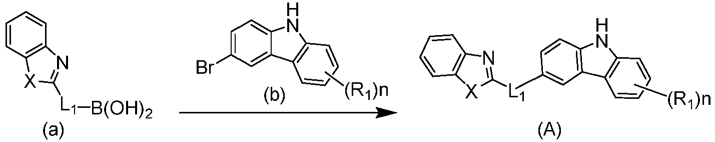

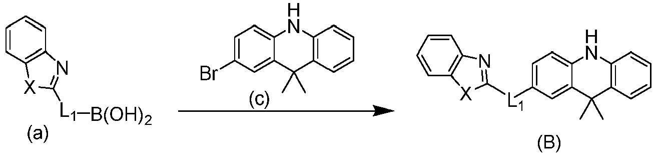

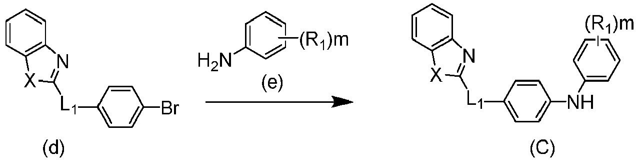

Compound (a) is subjected to a Suzuki reaction with Compound (b) to obtain Intermediate (A), or Compound (a) is subjected to a Suzuki reaction with Compound (c) to obtain Intermediate (B), or Compound (d) is subjected to a Buchwald reaction with Compound (e) to obtain Intermediate (C). Intermediate (A), (B) or (C) is then subjected to a Buchwald reaction with a halide (f) Br-L-Br or I-L-Br containing an L group, to finally obtain a target compound represented by Formula (I).

Wherein, the definitions of L, L₁, X, R₁, m and n are as described above, which will not be repeated herein.

Conditions for each reaction described above are not particularly restricted herein, and any reaction conditions known to those skilled in the art can be used. Sources of raw materials used in each reaction described above are not particularly restricted herein, and the raw materials may be commercially available products or may be prepared with a preparation method known to those skilled in the art. The heterocyclic compound in the present disclosure requires fewer synthetic steps and is simple in treatment, and thus is suitable for industrial production.

The present disclosure further provides an organic electroluminescent device comprising the heterocyclic compound. Preferably, the organic electroluminescent device includes a cover layer, a cathode, an organic layer, an anode and a substrate, wherein the cover layer contains the heterocyclic compound.

For the organic electroluminescent device of the present disclosure, the organic layer may include a hole injection layer, a hole transport layer, a light emitting layer, an electron transport layer and an electron injection layer. However, the structure of the organic electroluminescent device in the present disclosure is not limited to the above structure, and if necessary, the organic layer may be omitted or there may be multiple organic layers simultaneously. For example, an electron blocking layer may further be provided between the hole transport layer and the light emitting layer, and a hole blocking layer may further be provided between the electron transport layer and the light emitting layer. Organic layers having the same function may be made into a laminate structure with more than two layers. For example, the hole transport layer may further include a first hole transport layer and a second hole transport layer, and the electron transport layer may further include a first electron transport layer and a second electron transport layer.

As regards the organic electroluminescent device in the present disclosure, except the cover layer which contains the heterocyclic compound represented by Formula (I), other layers may use any material in the existing art useful for such layers.

As regards the anode of the organic electroluminescent device in the present disclosure, a material having a high work function may be used, for example, a metal such as vanadium, chromium, copper, zinc, gold or an alloy thereof; a metal oxide such as zinc oxide, indium oxide, indium tin oxide (ITO) and indium zinc oxide (IZO); a combination of a metal and an oxide, such as ZnO:Al or SnO₂:Sb; and a conductive polymer such as poly(3-methyl compound), poly[3,4-(ethylene-1,2-dioxy)thiophene] (PEDOT), polypyrrole, polyaniline and the like, but is not limited thereto.

As regards the cathode of the organic electroluminescent device in the present disclosure, a material having a low work function may be used, for example, a metal such as magnesium, calcium, sodium, potassium, titanium, indium, yttrium, lithium, gadolinium, aluminum, silver, tin, lead or an alloy thereof; and a material with a multilayer-structure, such as LiF/Al or LiO₂/Al, but is not limited thereto.

As regards the hole transport layer of the organic electroluminescent device in the present disclosure, the material of the hole transport layer is required to have a proper ionic potential and a large hole mobility, and may be an organic material based on arylamine, a conductive polymer, a block copolymer with both conjugated and non-conjugated parts, but is not limited thereto. Examples include 4,4'-bis[N-(1-naphthyl)-N-phenylamino]biphenyl (NPB), 4,4,4"-tris(N-carbazolyl)triphenylamine (TCTA), N,N'-dis(3-methylphenyl)-N,N'-dibenzyl-[1,1-biphenyl]-4,4'-diamine (TPD), N,N'-dis(naphthalen-1-yl)-N,N'-diphenylbenzidine (a-NPD), or the like.

As regards the light emitting layer of the organic electroluminescent device in the present disclosure, the light emitting material may be a red light emitting material, a green light emitting material, or a blue light emitting material, and if necessary, two or more light emitting materials may be mixed. In addition, the light emitting material may be only a host material, or may be the mixture of a host material and a doped material. Preferably, the light emitting layer uses the mixture of a host material and a doped material.

As regards the electron transport layer of the organic electroluminescent device in the present disclosure, the electron transport material is required to have a high electron affinity, and be able to effectively transport electrons, so that substances such as impurities that will become traps are not easily produced during manufacture and use. The electron transport material used in the electron transport layer is not particularly limited, which may be, but is not limited to, (1) metal complexes such as aluminum complexes, beryllium complexes and zinc complexes, (2) aromatic heterocyclic compounds such as imidazole derivatives, benzimidazole derivatives and phenanthroline derivatives, and (3) polymer compounds. For example, the electron transport material described above may be tris(8-hydroxyquinoline)aluminum (Alq₃), 1,3,5-tris(1-naphthyl-1H-benzoimidazol-2-yl)benzene (TPBI), 4,7-diphenyl-1,10-phenanthroline (BPhen) and other phenanthroline derivatives, and the like.

The main function of the electron injection layer of the organic electroluminescent device in the present disclosure is to improve the efficiency of electron injection into the electron transport layer and the light emitting layer from the cathode, and thus the electron injection layer is required to be capable of transmitting electrons. The material of the electron injection layer may be salts of alkali metal such as lithium fluoride and cesium fluoride, salts of alkaline-earth metal such as magnesium fluoride, and metal oxides such as aluminum oxide, and the like.

Preferably, the cover layer includes a first cover layer containing the heterocyclic compound, and a second cover layer containing a compound represented by Formula (V). 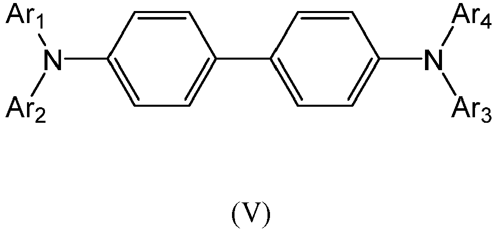 wherein Ar₁ to Ar₄ are each independently selected from any one of substituted or unsubstituted C6 - C30 aryl.

Preferably, Ar₁ to Ar₄ are each independently selected from any one of the following groups:

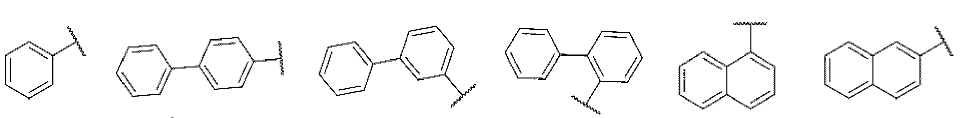

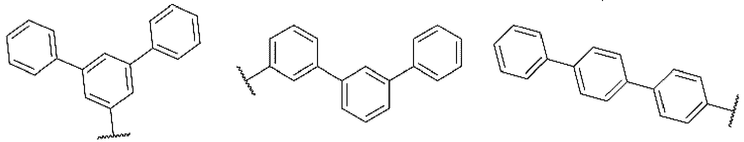

Preferably, the compound represented by Formula (V) is selected from any one of the following structures:

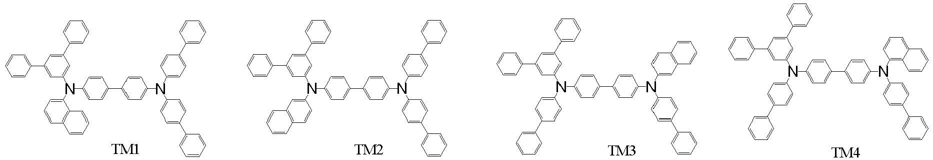

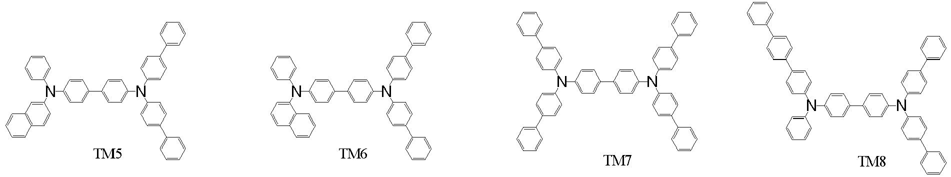

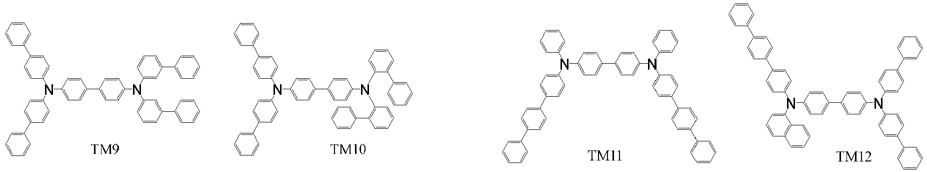

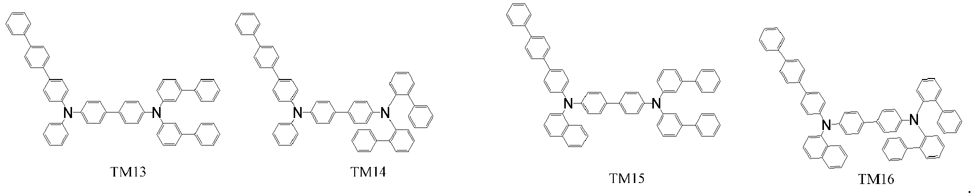

Preferably, the organic layer includes a hole injection layer including a host material and a doped material, wherein the host material has a structure represented by Formula (VI): 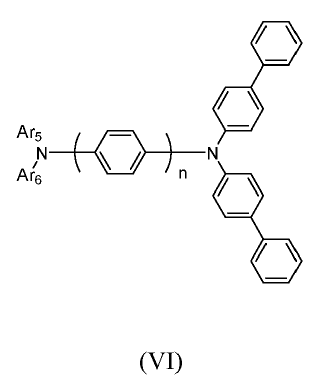 wherein n is 1, 2 or 3, and Ar₅ and Ar₆ are each independently selected from any one of substituted or unsubstituted C6 - C30 aryl.

Preferably, Ar₅ and Ar₆ are each independently selected from any one of the following groups:

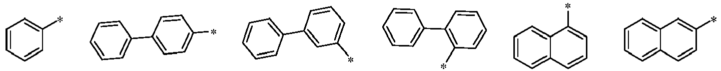

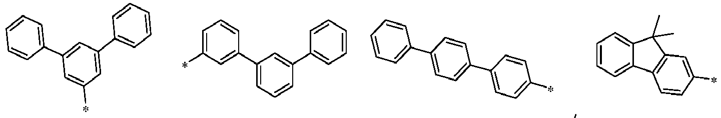

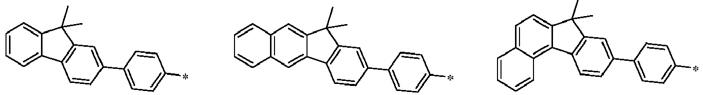

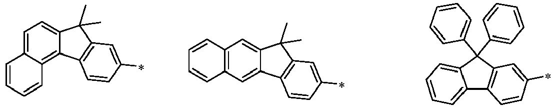

The doped material has a structure represented by Formula (VII): 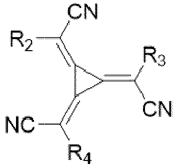 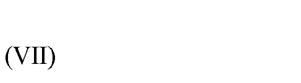 wherein R₂ to R₄ are each independently selected from any one of the following groups:

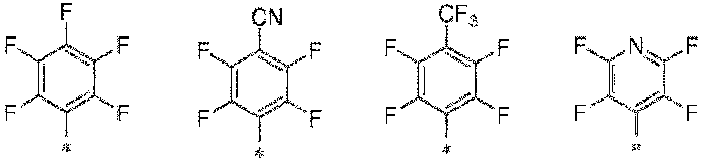

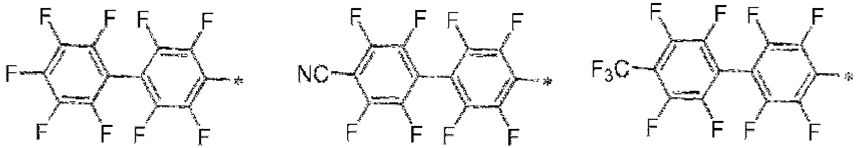

Preferably, the compound represented by Formula (VI) is selected from any one of the following structures:

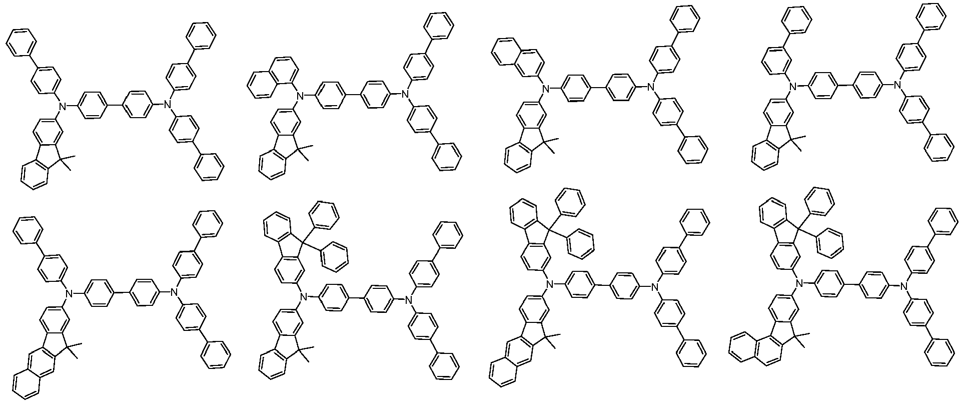

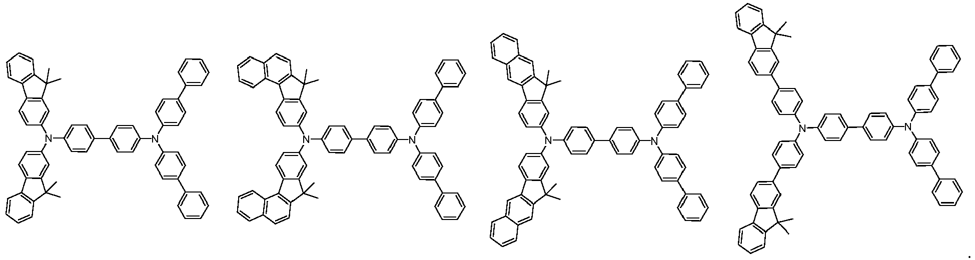

The thickness of each organic layer of the organic electroluminescent device is not particularly restricted herein, and any thickness known to those skilled in the art can be used.

The organic electroluminescent device in the present disclosure can be prepared in various ways, such as a solution coating method including spin coating, ink jet printing and the like, or a vacuum evaporation method.

Sources of raw materials used in examples described below are not particularly restricted herein, and the raw materials may be commercially available products or may be prepared with a preparation method known to those skilled in the art.

The mass spectrum of the compounds of the present disclosure is analyzed using the AXIMA-CFR plus Matrix-assisted Laser Desorption/Ionization Time-of-Flight Mass Spectrometry available from Kratos Analytical (UK) of Shimadzu Co., with chloroform as the solvent.

The element analysis is performed using Vario EL cube Elemental Analyzer available from Elementar Co., Germany, with sample mass of 5 mg.

The nuclear magnetic resonance (¹H NMR) is performed using the Bruker-510 nuclear magnetic resonance spectrometer available from Bruker Co., Germany, in 600 MHz, with CDCl₃ as the solvent and TMS as internal standard.

### Example 1 Preparation of Compound 9

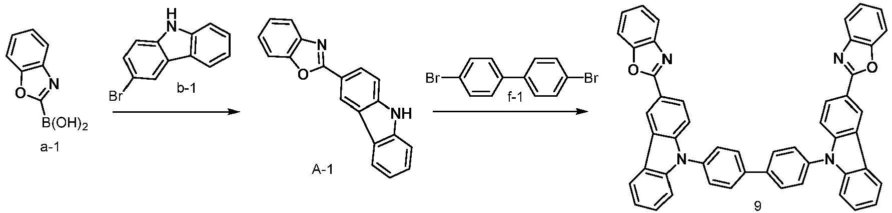

Under nitrogen protection, Compound a-1 (4.89 g, 30 mmol), Compound b-1 (7.46 g, 30.3 mmol), K₂CO₃ (12.44 g, 90 mmol) and 300 mL of toluene solvent were added to a 1L reaction flask and stirred. Catalyst Pd(PPh₃)₄ (0.35 g, 0.3 mmol) and 50 mL of distilled water were added to the flask. The reaction was heated to reflux and stirred for 10 hours. After full reaction, 75 mL of distilled water was added to terminate the reaction. The resultant was filtrated under reduced pressure to obtain crude Intermediate A-1, which was washed three times with distilled water, and recrystallized from toluene and ethanol (10: 1) to obtain 6.65 g of the target Compound A-1 in a yield of 78%.

Under argon atmosphere, Intermediate A-1 (5.74 g, 20.2 mmol), f-1 (3.12 g, 10 mmol) and sodium tert-butoxide (2.40 g, 25 mmol) were dissolved in 100 ml of dehydrated toluene, a solution of palladium acetate (0.04 g, 0.2 mmol) and tri-tert-butylphosphine (0.16 g, 0.8 mmol) in toluene was added with stirring, and the reaction was refluxed for 8 hours. After cooling, the mixture was filtrated through a celite/silica funnel. The filtrate was distilled under reduced pressure to remove the organic solvent. The concentrated solution was recrystallized from toluene and ethanol (14: 1) and then filtrated to obtain Compound 9 (4.96 g, 6.9 mmol) in a yield of 69%.

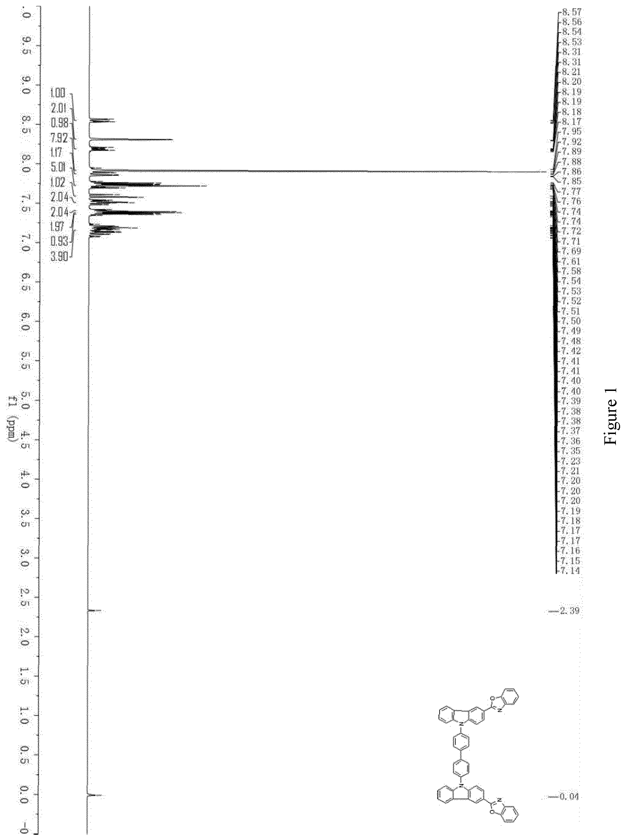

Mass spectrum (m/z): 718.40 (Calcd.: 718.24). Theoretical element content (%) of C₅₀H₃₀N₄O₂: C, 83.55; H, 4.21; N, 7.79; O, 4.45. Measured elemental content (%): C, 83.66; H, 4.20; N, 7.81; O, 4.48. ¹H NMR (500 MHz, CDCl₃): δ 8.55 (dd, 1H), 8.31 (d, 2H), 8.22 - 8.16 (m, 1H), 7.92 (s, 8H), 7.87 (dd, 1H), 7.77 - 7.69 (m, 5H), 7.59 (d, 1H), 7.51 (m, 2H), 7.41 (m, 2H), 7.38 (d, 2H), 7.36 (d, 1H), 7.22 - 7.09 (m, 4H). FIG. 1 is a ¹H NMR diagram of Compound 9 prepared in Example 1 of the present disclosure. The above results confirmed that the obtained product was the target product.

### Example 2 Preparation of Compound 12

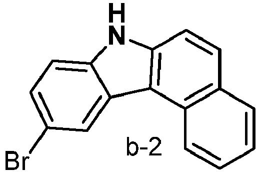

Compound 12 was obtained according to the same procedures as Example 1 except that an equal molar amount of bromide b-2 was used in place of bromide b-1. Mass spectrum (m/z): 818.39 (Calcd.: 818.27). Theoretical element content (%) of C₅₈H₃₄N₄O₂: C, 85.07; H, 4.18; N, 6.84; O, 3.91. Measured elemental content (%): C, 85.14; H, 4.21; N, 6.89; O, 3.90. The above results confirmed that the obtained product was the target product.

### Example 3 Preparation of Compound 16

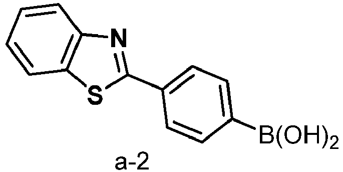

Compound 16 was obtained according to the same procedures as Example 1 except that an equal molar amount of a-2 was used in place of a-1. Mass spectrum (m/z): 902.40 (Calcd.: 902.25). Theoretical element content (%) of C₆₂H₃₈N₄S₂: C, 82.46; H, 4.24; N, 6.20; S, 7.10. The measured elemental content (%): C, 82.54; H, 4.24; N, 6.24; S, 7.12. The above results confirmed that the obtained product was the target product.

### Example 4 Preparation of Compound 18

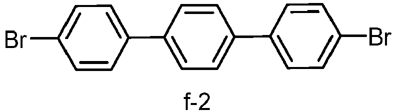

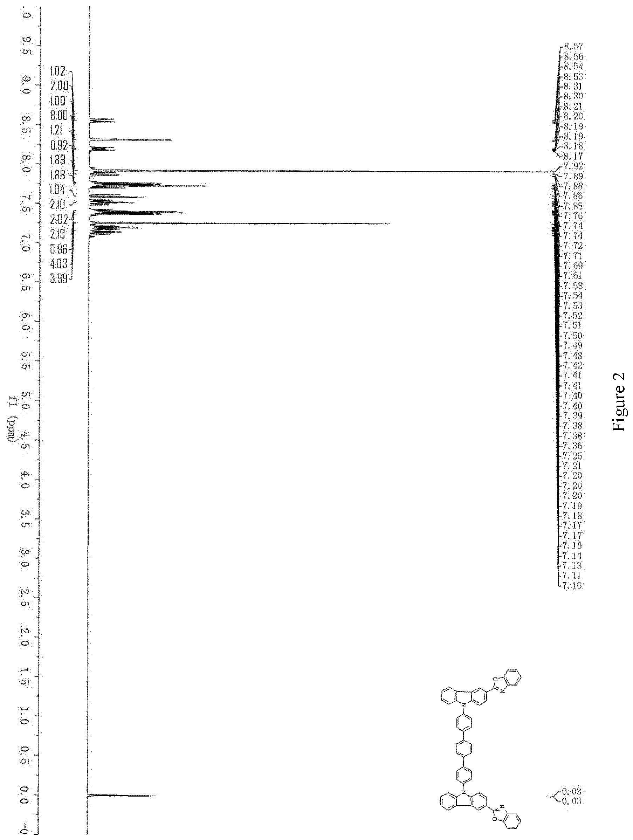

Compound 18 was obtained according to the same procedures as Example 1 except that an equal molar amount of f-2 was used in place of f-1. Mass spectrum (m/z): 794.38 (Calcd.: 794.27). Theoretical element content (%) of C₅₆H₃₄N₄O₂: C, 84.61; H, 4.31; N, 7.05; O, 4.03. Measured elemental content (%): C, 84.70; H, 4.32; N, 7.08; O, 4.04. ¹H NMR (500 MHz, CDCl₃): δ 8.55 (dd, 1H), 8.31 (d, 2H), 8.22 - 8.16 (m, 1H), 7.92 (s, 8H), 7.87 (dd, 1H), 7.76 (s, 1H), 7.74 (d, 2H), 7.71 (d, 2H), 7.59 (d, 1H), 7.51 (m, 2H), 7.41 (m, 2H), 7.38 (d, 2H), 7.36 (s, 1H), 7.25 (s, 4H), 7.22 - 7.10 (m, 4H). FIG. 2 is a ¹H NMR diagram of Compound 18 prepared in Example 4 of the present disclosure. The above results confirmed that the obtained product was the target product.

### Example 5 Preparation of Compound 24

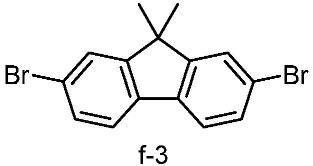

Compound 24 was obtained according to the same procedures as Example 1 except that an equal molar amount of f-3 was used in place of f-1. Mass spectrum (m/z): 758.40 (Calcd.: 758.27). Theoretical element content (%) of C₅₃H₃4N₄O₂: C, 83.88; H, 4.52; N, 7.38; O, 4.22. Measured elemental content (%): C, 83.99; H, 4.52; N, 7.42; O, 4.24. The above results confirmed that the obtained product was the target product.

### Example 6 Preparation of Compound 27

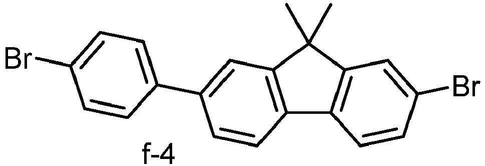

Compound 27 was obtained according to the same procedures as Example 1 except that an equal molar amount of f-4 was used in place of f-1. Mass spectrum (m/z): 834.45 (Calcd.: 834.30). Theoretical element content (%) of C₅₉H₃₈N₄O₂: C, 84.87; H, 4.59; N, 6.71; O, 3.83. Measured elemental content (%): C, 84.99; H, 4.60; N, 6.74; O, 3.84. The above results confirmed that the obtained product was the target product.

### Example 7 Preparation of Compound 34

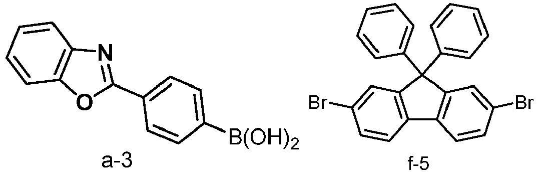

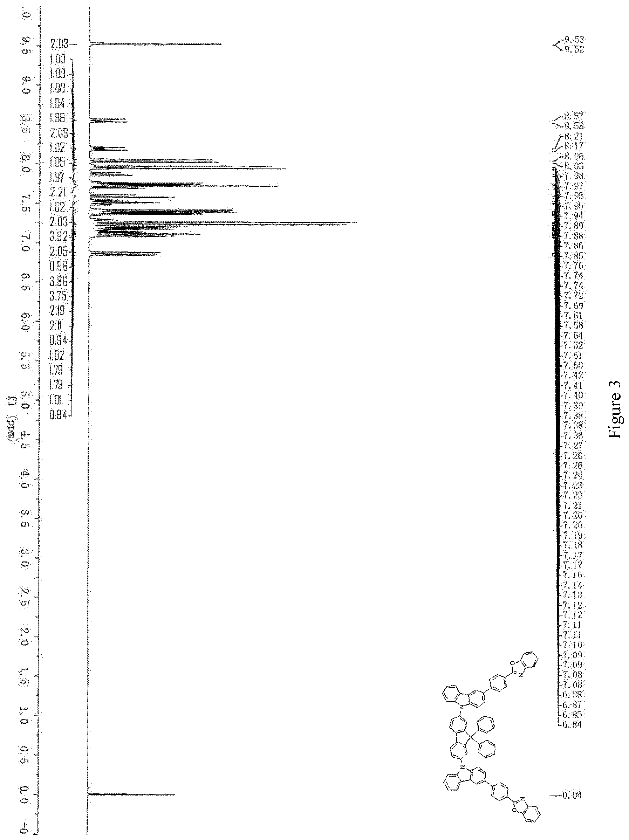

Compound 34 was obtained according to the same procedures as Example 1 except that an equal molar amount of a-3 was used in place of a-1 and that an equal molar amount of f-5 was used in place of f-1. Mass spectrum (m/z): 1034.49 (Calcd.: 1034.36). Theoretical element content (%) of C₇₅H₄₆N₄O₂: C, 87.02; H, 4.48; N, 5.41; O, 3.09. Measured elemental content (%): C, 87.14; H, 4.50; N, 5.42; O, 3.10. ¹H NMR (500 MHz, CDCl₃): δ 9.52 (d, 2H), 8.55 (d, 1H), 8.19 (d, 1H), 8.06 (s, 1H), 8.03 (s, 1H), 7.97 (d, 2H), 7.96 - 7.92 (m, 2H), 7.87 (dd, 1H), 7.76 (s, 1H), 7.74 (d, 2H), 7.71 (d, 2H), 7.59 (d, 1H), 7.52 (dd, 2H), 7.40 (dd, 4H), 7.38 (d, 2H), 7.36 (s, 1H), 7.27 (m, 4H), 7.25 - 7.22 (m, 4H), 7.20 (dd, 2H), 7.17 (dd, 2H), 7.13 (d, 1H), 7.12 (d, 1H), 7.11 (m, 2H), 7.09 (dd, 2H), 6.88 (d, 1H), 6.85 (d, 1H). FIG. 3 is a ¹H NMR diagram of Compound 34 prepared in Example 7 of the present disclosure. The above results confirmed that the obtained product was the target product.

### Example 8 Preparation of Compound 51 (not according to the invention)

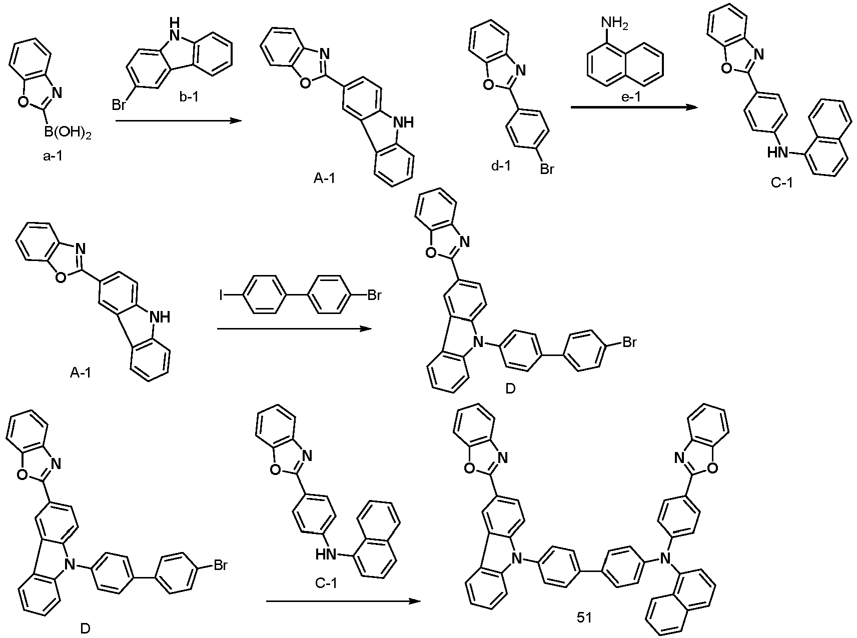

The synthesis of Intermediate A-1 was the same as that in Example 1.

Under argon atmosphere, Compound d-1 (4.11 g, 15 mmol), e-1 (2.16 g, 15.15 mmol) and sodium tert-butoxide (2.11 g, 22 mmol) were dissolved in 100 ml of dehydrated toluene, a solution of palladium acetate (0.03 g, 0.15 mmol) and tri-tert-butylphosphine (0.12 g, 0.6 mmol) in toluene was added with stirring, and the reaction was refluxed for 8 hours. After cooling, the mixture was filtrated through a celite/silica funnel. The filtrate was distilled under reduced pressure to remove the organic solvent. The residue was recrystallized from toluene and ethanol (10: 1) and then filtrated to obtain Intermediate C-1 (3.99 g, 11.85 mmol) in a yield of 79%.

Under argon atmosphere, Intermediate A-1 (4.11 g, 18 mmol), 4-bromo-4'-iodobiphenyl (2.16 g, 18.2 mmol) and sodium tert-butoxide (3.36 g, 35 mmol) were dissolved in 100 ml of dehydrated toluene, a solution of palladium acetate (0.038 g, 0.19 mmol) and tri-tert-butylphosphine (0.15 g, 0.76 mmol) in toluene was added with stirring, and the reaction was refluxed for 8 hours. After cooling, the mixture was filtrated through a celite/silica funnel. The filtrate was distilled under reduced pressure to remove the organic solvent. The concentrated solution was recrystallized from toluene and ethanol (12: 1) and then filtrated to obtain Intermediate D (6.85 g, 13.3 mmol) in a yield of 74%.

Under argon atmosphere, Intermediate D (5.15 g, 10 mmol), Intermediate C-1 (3.53 g, 10.5 mmol) and sodium tert-butoxide (2.88 g, 30 mmol) were dissolved in 100 ml of dehydrated toluene, a solution of palladium acetate (0.03 g, 0.15 mmol) and tri-tert-butylphosphine (0.12 g, 0.6 mmol) in toluene was added with stirring, and the reaction was refluxed for 8 hours. After cooling, the mixture was filtrated through a celite/silica funnel. The filtrate was distilled under reduced pressure to remove the organic solvent. The concentrated solution was recrystallized from toluene and ethanol (14: 1) and then filtrated to obtain Compound 51 (5.01 g, 6.5 mmol) in a yield of 65%.

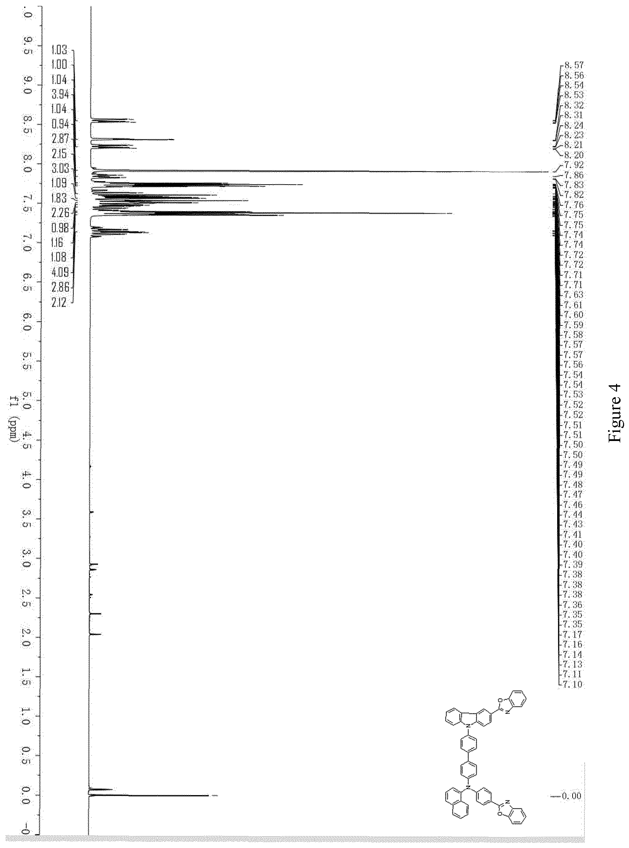

Mass spectrum (m/z): 770.39 (Calcd.: 770.27). Theoretical element content (%) of C₅₄H₃₄N₄O₂: C, 84.14; H, 4.45; N, 7.27; O, 4.15. Measured elemental content (%): C, 84.26; H, 4.44; N, 7.29; O, 4.16. ¹H NMR (500 MHz, CDCl₃): δ 8.55 (dd, 1H), 8.31 (d, 1H), 8.22 (dd, 1H), 7.92 (s, 4H), 7.87 - 7.81 (m, 1H), 7.76 (s, 1H), 7.74 (dd, 3H), 7.72 (d, 2H), 7.67 - 7.57 (m, 3H), 7.56 (d, 1H), 7.54 (m, 2H), 7.51 (m, 2H), 7.49 - 7.47 (m, 1H), 7.45 (dd, 1H), 7.40 (s, 1H), 7.38 (dd, 4H), 7.35 (m, 3H), 7.13 (m, 2H). FIG. 4 is a ¹H NMR diagram of Compound 51 prepared in Example 8 of the present disclosure. The above results confirmed that the obtained product was the target product.

### Example 9 Preparation of Compound 95

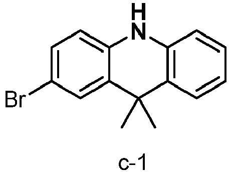

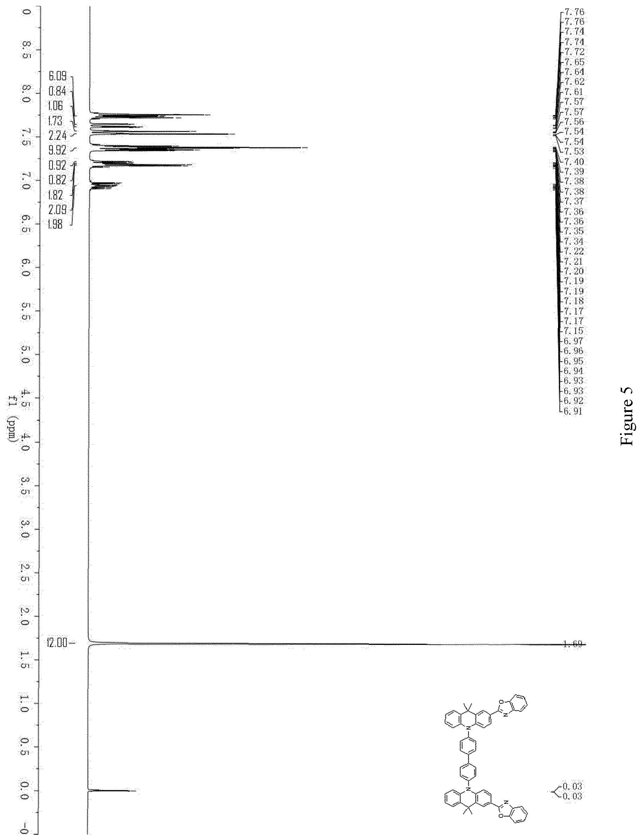

Compound 95 was obtained according to the same procedures as Example 1 except that an equal molar amount of c-1 was used in place of b-1. Mass spectrum (m/z): 802.46 (Calcd.: 802.33). Theoretical element content (%) of C₅₆H₄₂N₄O₂: C, 83.77; H, 5.27; N, 6.98; O, 3.98. Measured elemental content (%): C, 83.90; H, 5.29; N, 7.00; O, 3.99. ¹H NMR (500 MHz, CDCl₃): δ 7.78 - 7.70 (m, 6H), 7.65 (d, 1H), 7.62 (d, 1H), 7.58 - 7.55 (m, 2H), 7.55 - 7.53 (m, 2H), 7.40 - 7.34 (m, 10H), 7.22 (d, 1H), 7.20 (d, 1H), 7.19 (d, 2H), 7.17 (d, 2H), 6.94 (m, 2H), 1.69 (s, 12H). FIG. 5 is a ¹H NMR diagram of Compound 9 prepared in Example 9 of the present disclosure. The above results confirmed that the obtained product was the target product.

### Example 10 Preparation of Compound 99

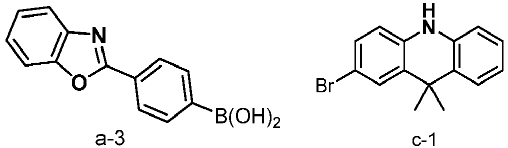

Compound 99 was obtained according to the same procedures as Example 1 except that an equal molar amount of a-3 was used in place of a-1 and that an equal molar amount of c-1 was used in place of b-1. Mass spectrum (m/z): 954.51 (Calcd.: 954.39). Theoretical element content (%) of C₆₈H₅₀N₄O₂: C, 85.51; H, 5.28; N, 5.87; O, 3.35. Measured elemental content (%): C, 85.60; H, 5.29; N, 5.90; O, 3.36. The above results confirmed that the obtained product was the target product.

### Example 11 Preparation of Compound 102

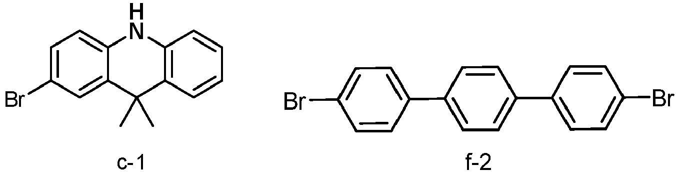

Compound 102 was obtained according to the same procedures as Example 1 except that an equal molar amount of c-1 was used in place of b-1 and that an equal molar amount of f-2 was used in place of f-1. Mass spectrum (m/z): 878.49 (Calcd.: 878.36). Theoretical element content (%) of C₆₂H₄₆N₄O₂: C, 84.71; H, 5.27; N, 6.37; O, 3.64. Measured elemental content (%): C, 84.82; H, 5.29; N, 6.40; O, 3.65. The above results confirmed that the obtained product was the target product.

### Example 12 Preparation of Compound 106

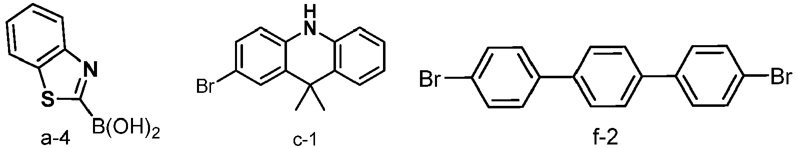

Compound 106 was obtained according to the same procedures as Example 1 except that an equal molar amount of a-4 was used in place of a-1, an equal molar amount of c-1 was used in place of b-1, and an equal molar amount of f-2 was used in place of f-1. Mass spectrum (m/z): 910.48 (Calcd.: 910.32). Theoretical element content (%) of C₆₂H₄₆N₄S₂: C, 81.73; H, 5.09; N, 6.15; S, 7.04. Measured elemental content (%): C, 81.80; H, 5.10; N, 6.16; S, 7.05. The above results confirmed that the obtained product was the target product.

### Example 13 Preparation of Compound 108

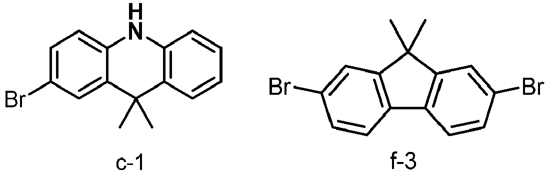

Compound 108 was obtained according to the same procedures as Example 1 except that an equal molar amount of c-1 was used in place of b-1 and that an equal molar amount of f-3 was used in place of f-1. Mass spectrum (m/z): 842.49 (Calcd.: 842.36). Theoretical element content (%) of C₅₉H₄₆N₄O₂: C, 84.06; H, 5.50; N, 6.65; O, 3.80. Measured elemental content (%): C, 84.14; H, 5.51; N, 6.66; O, 3.81. The above results confirmed that the obtained product was the target product.

### Example 14 Preparation of Compound 111

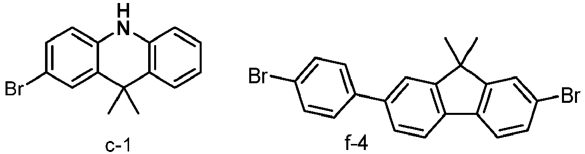

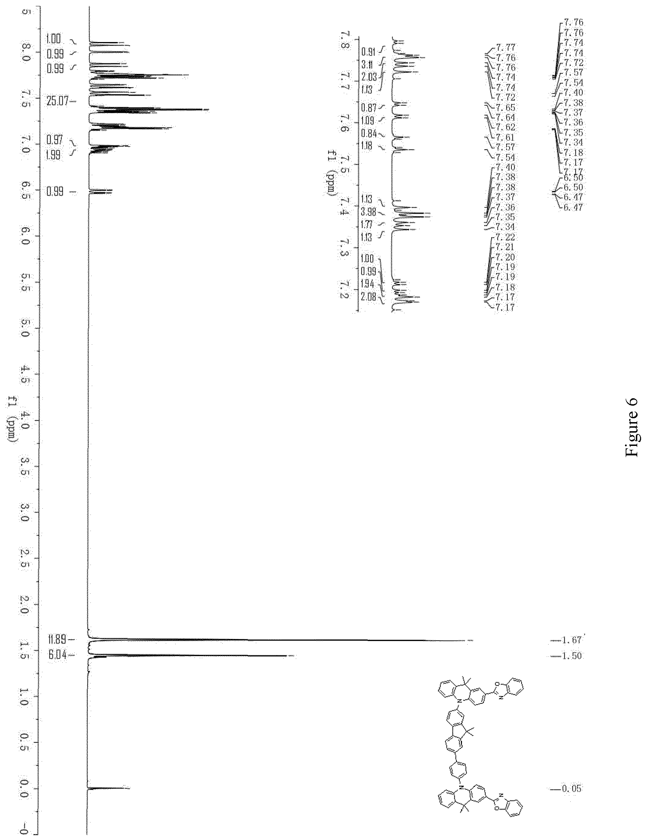

Compound 111 was obtained according to the same procedures as Example 1 except that an equal molar amount of c-1 was used in place of b-1 and that an equal molar amount of f-4 was used in place of f-1. Mass spectrum (m/z): 918.50 (Calcd.: 918.39). Theoretical element content (%) of C₆₅H₅₀N₄O₂: C,84.94; H, 5.48; N,6.10; O, 3.48. Measured elemental content (%): C, 85.02; H, 5.50; N, 6.13; O, 3.50. ¹H NMR (500 MHz, CDCl₃): δ 8.09 (d, 1H), 8.00 (d, 1H), 7.86 (d, 1H), 7.78 (dd, 1H), 7.76 (d, 3H), 7.74 (d, 2H), 7.72 (s, 1H), 7.65 (d, 1H), 7.62 (d, 1H), 7.56 (d, 1H), 7.55 - 7.52 (m, 1H), 7.40 (s, 1H), 7.39 - 7.37 (m, 4H), 7.36 (d, 2H), 7.34 (s, 1H), 7.23 - 7.21 (m, 1H), 7.20 (d, 1H), 7.19 (d, 2H), 7.17 (d, 2H), 6.98 (d, 1H), 6.94 (m, 2H), 6.48 (dd, 1H),1.67 (s, 12H), 1.50 (s, 6H). FIG. 6 is a ¹H NMR diagram of Compound 111 prepared in Example 14 of the present disclosure. The above results confirmed that the obtained product was the target product.

### Example 15 Preparation of Compound 114

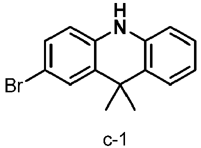

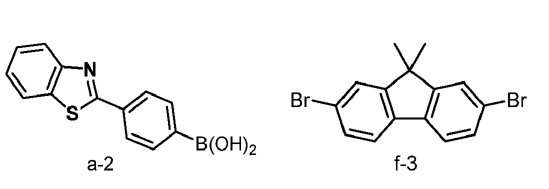

Compound 114 was obtained according to the same procedures as Example 1 except that an equal molar amount of a-2 was used in place of a-1, an equal molar amount of c-1 was used in place of b-1, and an equal molar amount of f-3 was used in place of f-1. Mass spectrum (m/z): 1026.56 (Calcd.: 1026.38). Theoretical element content (%) of C₇₁H₅₄N₄S₂: C, 83.01; H, 5.30; N, 5.45; S, 6.24. Measured elemental content (%): C, 83.13; H, 5.31; N, 5.49; S, 6.26. The above results confirmed that the obtained product was the target product.

### Example 16 Measurement of refractive index

Refractive index was measured at 620 nm using M-2000 spectroscopic ellipsometer available from J.A.Woollam, USA, wherein the scanning range of the instrument was 245 - 1000 nm, the size of the glass substrate was 200 × 200 mm, and the thickness of the material film was 60 nm. Results are shown in Table 1.

**Table 1 Refractive indexes (n) of compounds**

| Compound | Refractive Index (620 nm) |
|---|---|
| Compound 9 | 2.00 |
| Compound 12 | 1.98 |
| Compound 16 | 1.99 |
| Compound 18 | 2.01 |
| Compound 24 | 1.97 |
| Compound 27 | 1.96 |
| Compound 34 | 1.98 |
| Compound 51 | 1.95 |
| Compound 95 | 2.05 |
| Compound 99 | 2.06 |
| Compound 102 | 2.10 |
| Compound 106 | 2.07 |
| Compound 108 | 2.04 |
| Compound 111 | 2.02 |
| Compound 114 | 2.03 |

### Example 17 Measurement of glass transition temperature

Glass transition temperature was measured using DSC 821e differential scanning calorimeter available from Mettler-Toledo company, Switzerland, wherein the testing atmosphere was nitrogen, and the flow rate of the nitrogen was 200 ml/min; the scanning rate of the instrument was 10 °C/min, and the scanning range was 80 - 400°C; and the mass of the compound sample was 5 mg. The measurement results are shown in Table 2.

**Table 2 Glass transition temperature (Tg) of compounds**

| Compound | Glass transition temperature (°C) |
|---|---|
| Compound 9 | 135 |
| Compound 12 | 133 |
| Compound 16 | 134 |
| Compound 18 | 135 |
| Compound 24 | 133 |
| Compound 27 | 134 |
| Compound 34 | 131 |
| Compound 51 | 130 |
| Compound 95 | 140 |
| Compound 99 | 138 |
| Compound 102 | 136 |
| Compound 106 | 139 |
| Compound 108 | 136 |
| Compound 111 | 134 |
| Compound 114 | 137 |

### Application examples 1-11 Preparation of light emitting devices 1-11

First, on the ITO (10 nm)/Ag (100 nm)/ITO (10 nm) layer formed on an organic substrate, a hole injection layer (Compound HIL doped with 5% of Compound P-D, 25 nm), a hole transport layer (NPB, 60 nm), a light emitting layer (host CBP doped with 5% of (piq)₂Ir(acac), 30 nm), an electron transport layer (TPBI, 40 nm), and an electron injection layer (LiF, 0.5 nm) were deposited layer-by-layer on an ITO (10 nm)/Ag (100 nm)/ITO (10 nm) layer formed on an organic substrate. Then, Mg/Ag was deposited in a thickness of 20 nm to form a cathode. Finally, a heterocyclic compound in the present disclosure was deposited under vacuum in a thickness of 60 nm as a cover layer.

### Application examples 12 - 15 Preparation of light emitting devices 12 - 15

First, a hole injection layer (Compound HIL doped with 5% of Compound P-D, 25 nm), a hole transport layer (NPB, 60 nm), a light emitting layer (host CBP doped with 5% of (piq)₂Ir(acac), 30 nm), an electron transport layer (TPBI, 40 nm), and an electron injection layer (LiF, 0.5 nm) were deposited layer-by-layer on an ITO (10 nm)/Ag (100 nm)/ITO (10 nm) layer formed on an organic substrate. Then, Mg/Ag was deposited in a thickness of 20 nm to form a cathode. Finally, a heterocyclic compound in the present disclosure was deposited under vacuum in a thickness of 40 nm as a first cover layer, and a compound represented by Formula (V) was deposited under vacuum in a thickness of 20 nm as a second cover layer.

### Comparative examples 16-17 Preparation of light emitting devices 16-17

The difference of these examples from application examples 1 - 11 was that the cover layer was compound CP-1 or CP-2.

In the present disclosure, devices were prepared by using a vacuum evaporation system by continuously evaporating under a condition of uninterrupted vacuum. The materials used herein were separately placed in quartz crucibles under different evaporation sources, and the temperatures of the evaporation sources can be separately controlled. The thermal evaporation rates of organic materials or doped parent organic materials were generally set at 0.1 nm/s, and the evaporation rates of the doped materials were adjusted according to the doping ratio. The evaporation rates of electrode metals were 0.4 - 0.6 nm/s. The processed glass substrates were placed in an OLED vacuum coating machine. In the film manufacturing process, the vacuum degree of the system was maintained below 5 × 10⁻⁵ Pa. Organic layers and metal electrodes were respectively deposited by replacing mask plates. The evaporation rate was detected by an SQM160 quartz crystal film thickness detector from Inficon, and the film thickness was detected by a quartz crystal oscillator. Test software, computers, K2400 Digital Source Meter manufactured by Keithley, USA, and PR788 Spectrascan Photometer manufactured by Photo Research, USA were combined into a combined IVL test system to test the driving voltage, the light emitting efficiency and the CIE color coordinates of organic electroluminescent devices. The lifetime was tested using M6000 OLED Lifetime Test System from McScience Co. The environment for testing was atmospheric environment, and the temperature was room temperature.

The compounds involved in application examples and comparative examples of the present disclosure are shown below:

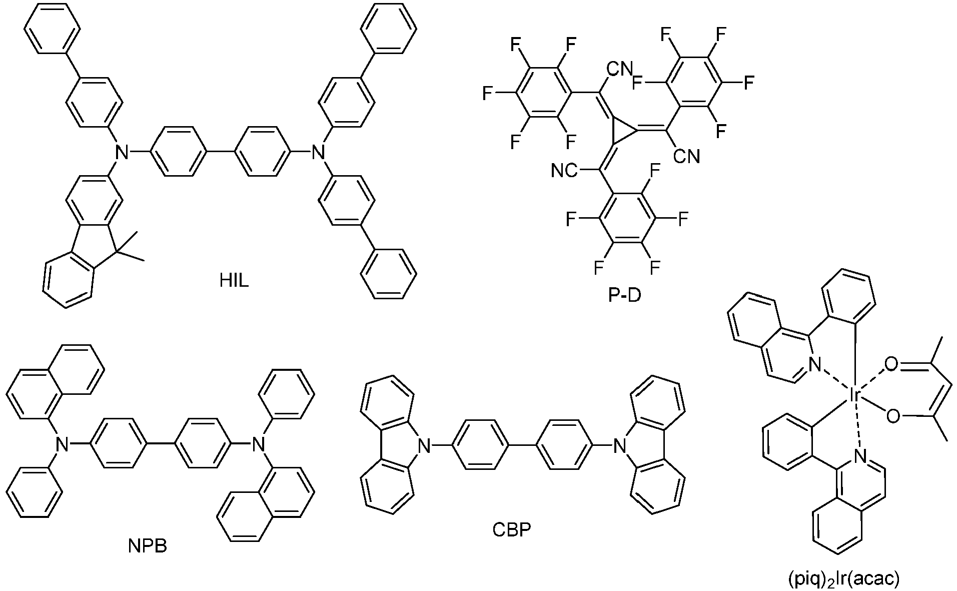

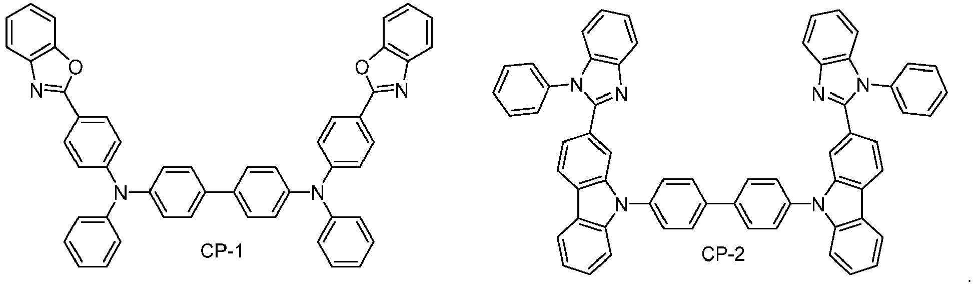

The light emitting properties of the organic electroluminescent devices prepared in the examples of the present disclosure are shown in Table 3.

**Table 3 Data on light emitting property test of organic electroluminescent devices**

| Example | First cover layer | Second cover layer | Driving voltage [V] (@20 mA/cm²) | Efficiency (cd/A) | Lifetime (%) |
|---|---|---|---|---|---|
| Application example 1 | Compound 9 | - | 4.00 | 22.1 | 124 |
| Application example 2 | Compound 16 | - | 4.01 | 21.8 | 126 |
| Application example 3 | Compound 18 | - | 4.00 | 22.0 | 127 |
| Application example 4 | Compound 27 | - | 3.99 | 21.6 | 125 |
| Application example 5 | Compound 34 | - | 3.97 | 21.5 | 122 |
| Application example 6 | Compound 95 | - | 4.02 | 22.9 | 132 |
| Application example 7 | Compound 99 | - | 4.00 | 22.5 | 130 |
| Application example 8 | Compound 102 | - | 3.98 | 22.7 | 133 |
| Application example 9 | Compound 106 | - | 4.01 | 22.8 | 131 |
| Application example 10 | Compound 108 | - | 3.98 | 22.5 | 128 |
| Application example 11 | Compound 114 | - | 4.00 | 22.3 | 129 |
| Application | Compound 18 | Compound | 4.01 | 22.0 | 123 |
| example 12 | | TM6 | | | |
| Application example 13 | Compound 95 | Compound TM7 | 3.97 | 22.6 | 134 |
| Application example 14 | Compound 24 | Compound TM8 | 4.02 | 22.3 | 125 |
| Application example 15 | Compound 111 | Compound TM7 | 4.02 | 22.5 | 130 |
| Comparative example 1 | Compound CP-1 | - | 4.00 | 18.0 | 104 |
| Comparative example 2 | Compound CP-2 | - | 3.99 | 17.1 | 100 |

The results show that when the heterocyclic compound in the present disclosure is used in an organic electroluminescent device as a cover layer, it can improve the light emitting efficiency and service life of the device, and thus is an organic light emitting material with excellent performance.

## Claims

1. A heterocyclic compound having a structure represented by Formula (I):
A-L-B Formula (I)
wherein L is selected from any one of the following groups: 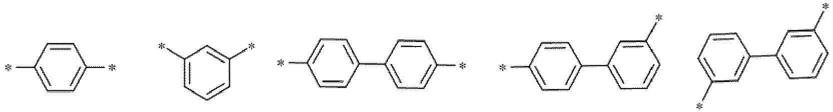 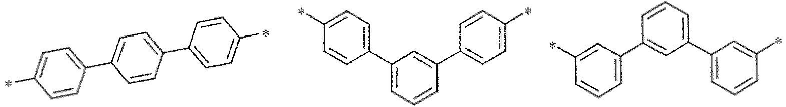 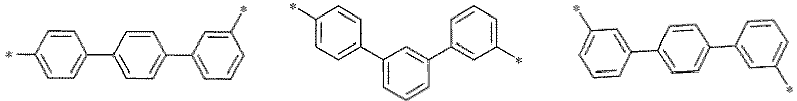 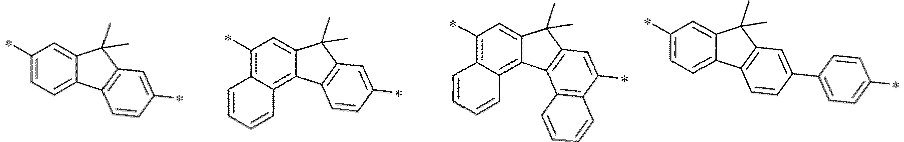 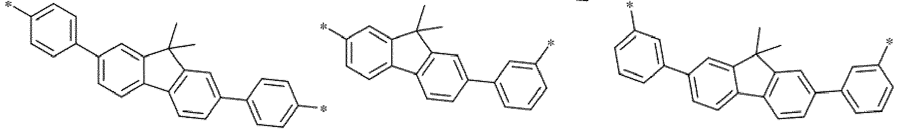 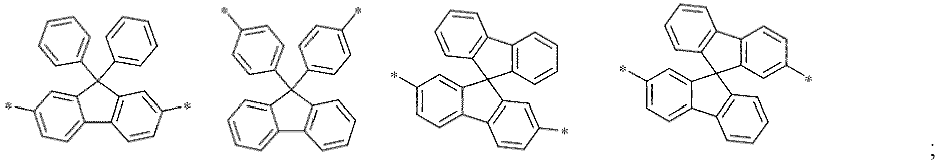 and
A and B are each independently selected from any one of Formula (II) or Formula (III): 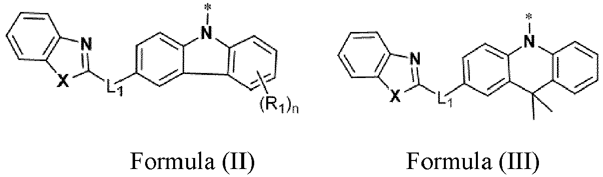
wherein X is O or S;
L₁ is a single bond, phenyl or biphenyl;
n is 0, 1, 2, 3 or 4; and
R₁ is H, C1 - C15 alkyl or C6 - C30 aryl, or two adjacent R₁ groups are bonded to form a ring structure.

2. The heterocyclic compound of claim 1, wherein A and B are independently selected from any one of the following formulas:

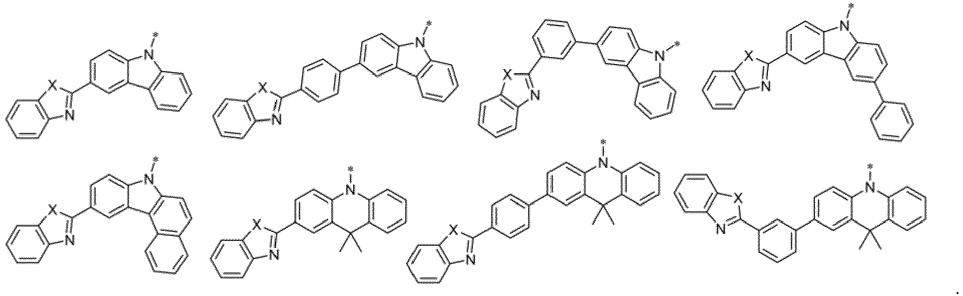

3. The heterocyclic compound of claim 1, wherein the heterocyclic compound is selected from any one of the following compounds:

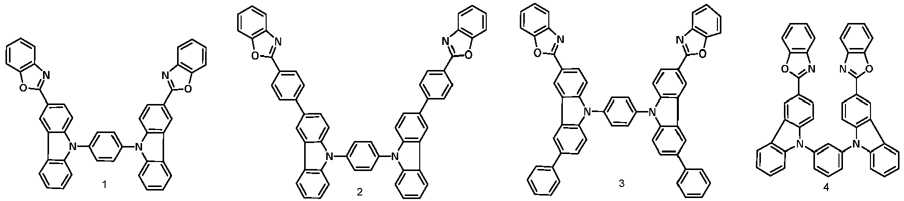

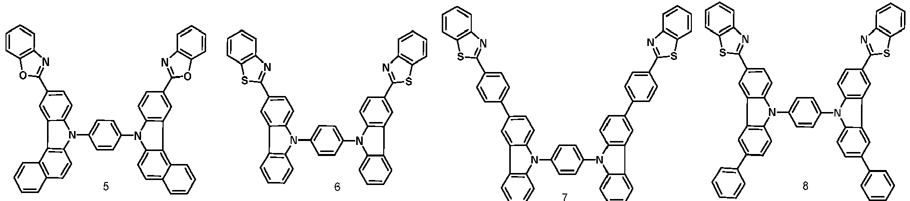

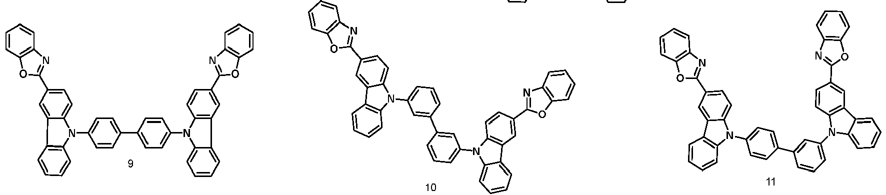

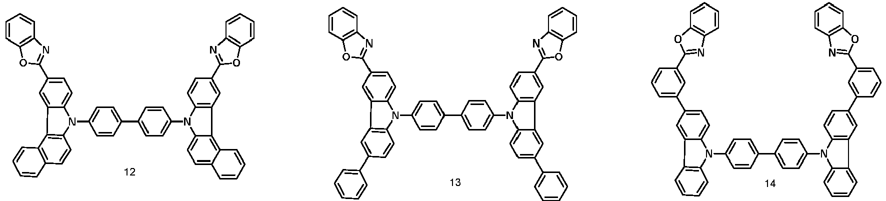

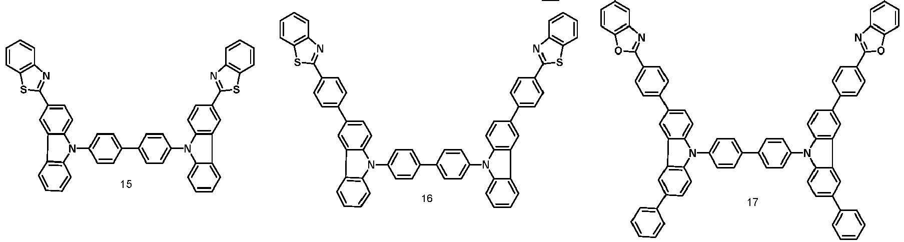

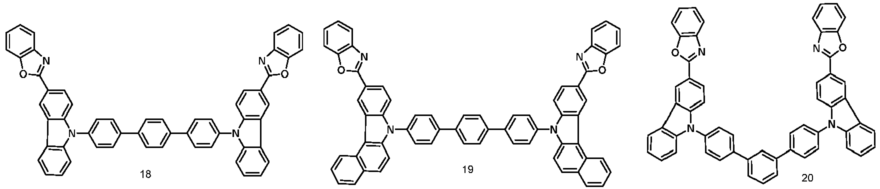

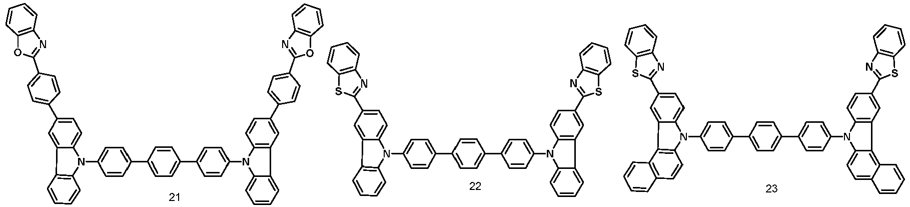

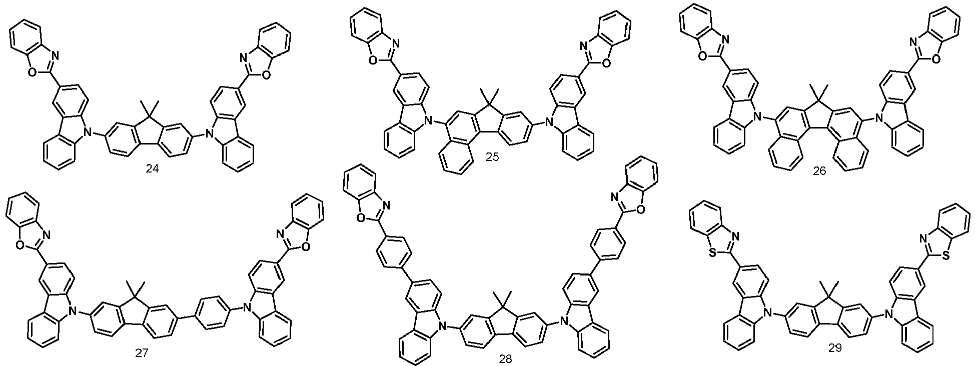

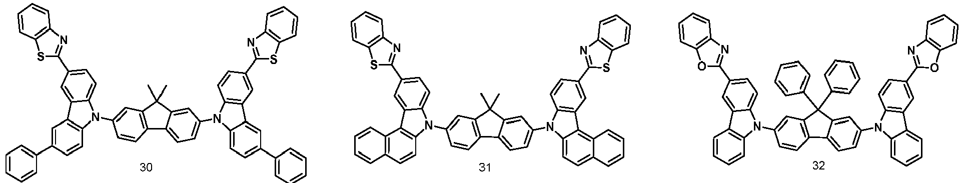

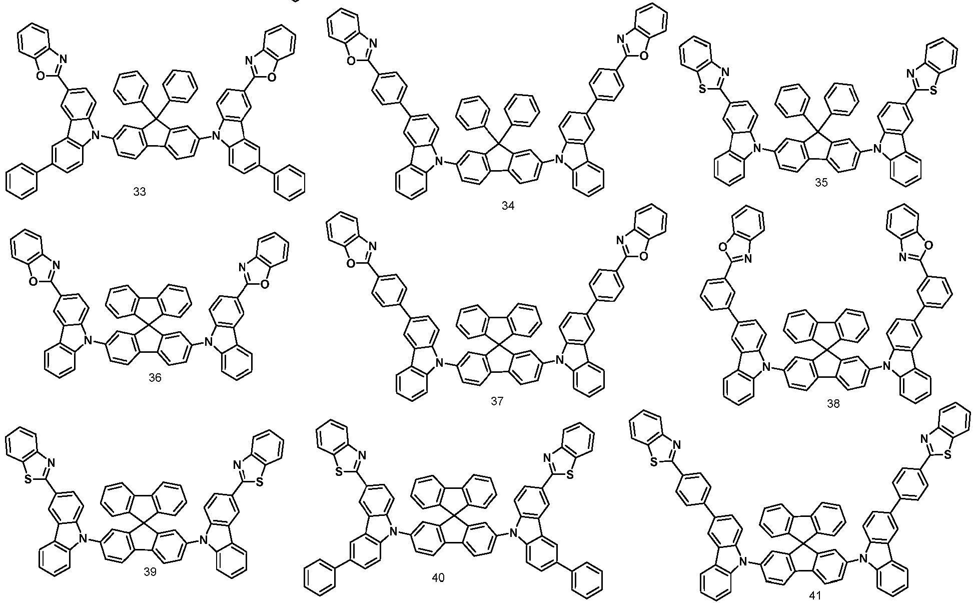

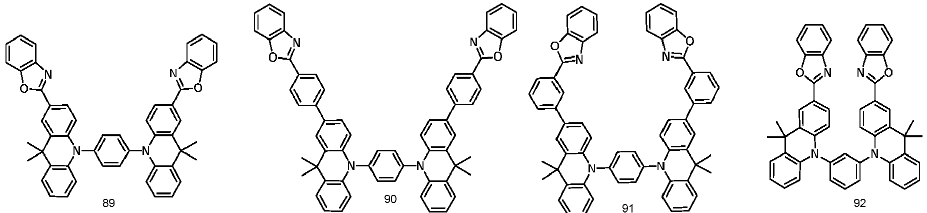

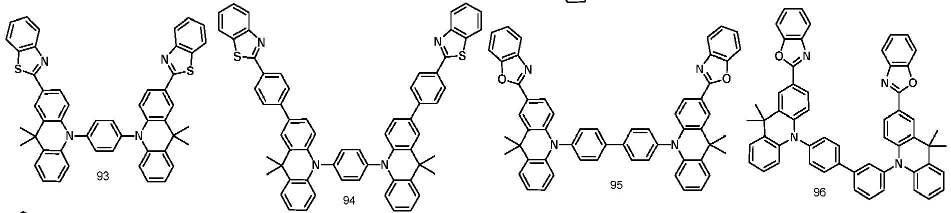

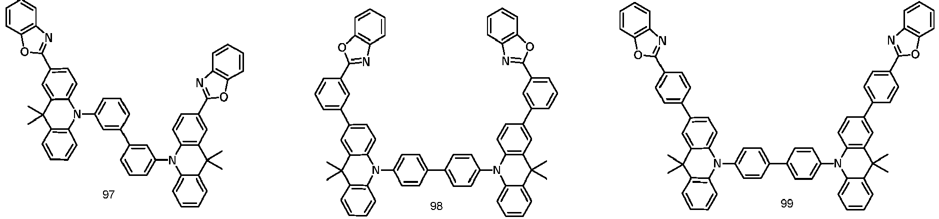

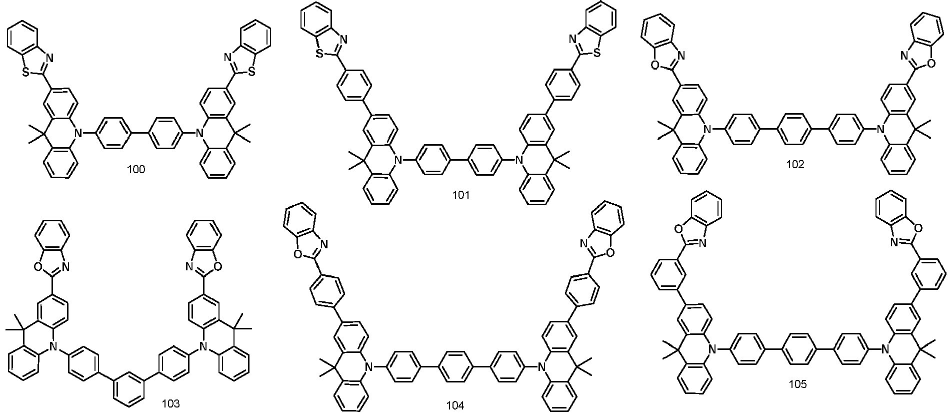

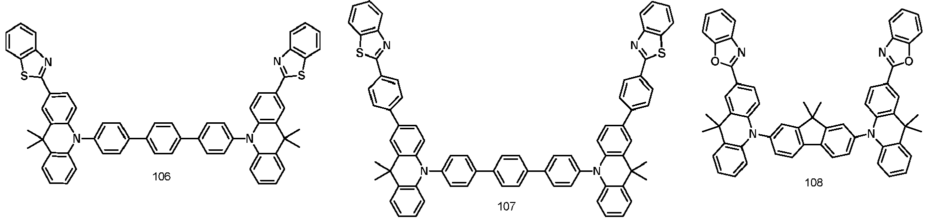

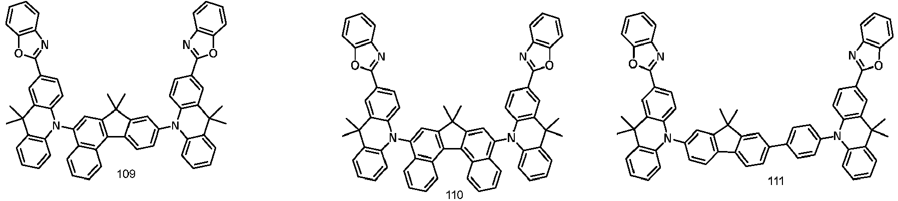

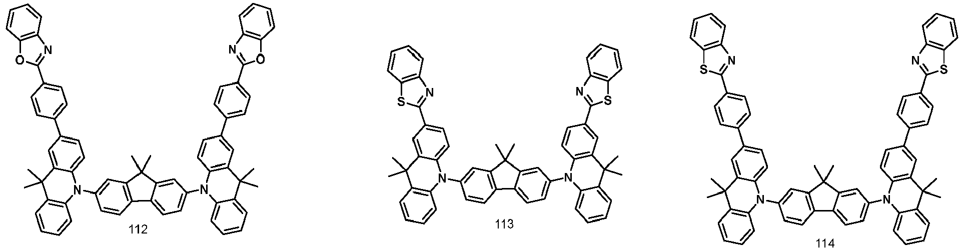

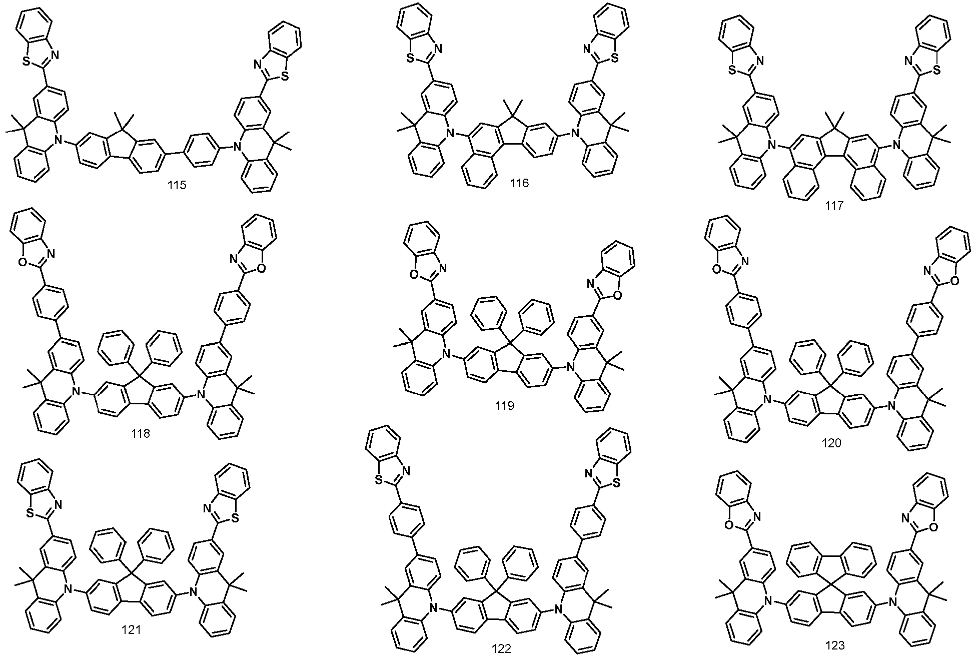

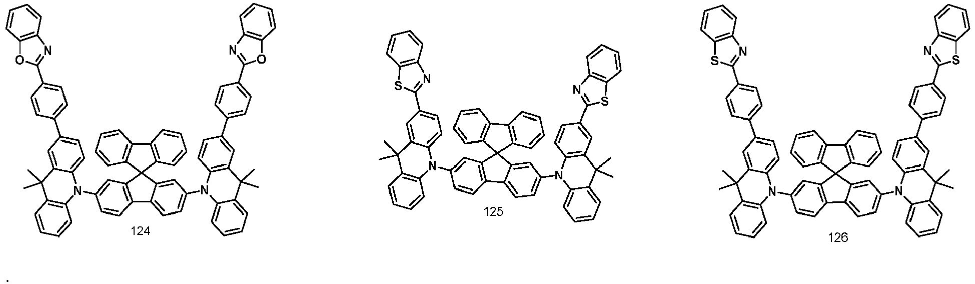

4. An organic electroluminescent device, comprising the heterocyclic compound of any one of claims 1 to 3.

5. The organic electroluminescent device of claim 4, wherein the organic electroluminescent device comprises a cover layer, a cathode, an organic layer, an anode and a substrate, wherein the cover layer contains the heterocyclic compound of any one of claims 1 to 3.

6. The organic electroluminescent device of claim 5, wherein the cover layer comprises a first cover layer and a second cover layer, wherein the first cover layer contains the heterocyclic compound of any one of claims 1 to 3, and the second cover layer contains a compound represented by Formula (V): 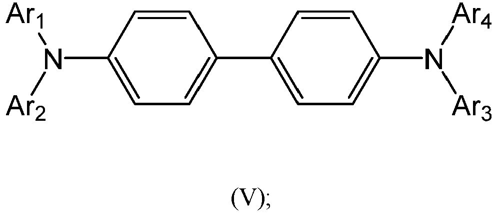 wherein Ar₁ to Ar₄ are each independently selected from any one of substituted or unsubstituted C6 - C30 aryl.

7. The organic electroluminescent device of claim 6, wherein the compound represented by Formula (V) is selected from any one of the following structures:

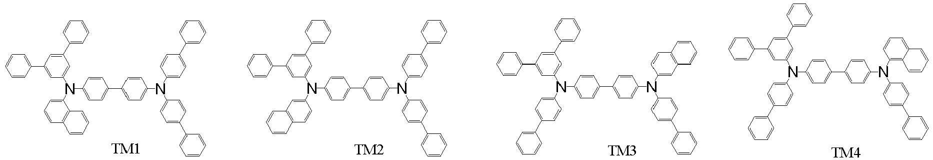

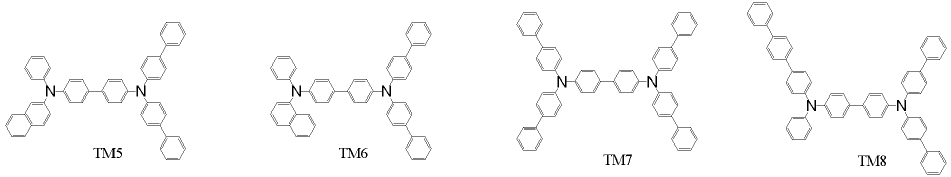

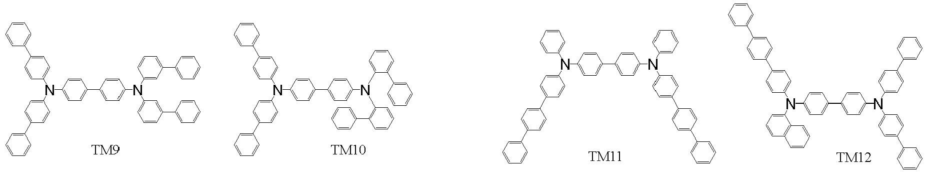

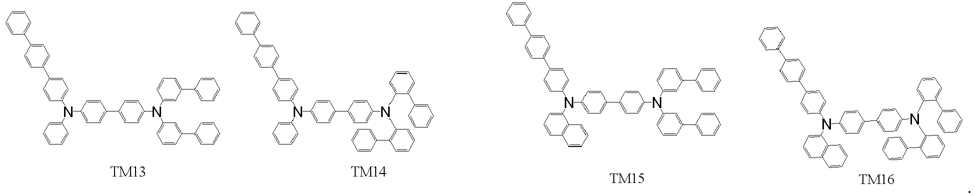

8. The organic electroluminescent device of claim 5, wherein the organic layer comprises a hole injection layer comprising a host material and a doped material, wherein the host material has a structure represented by Formula (VI): 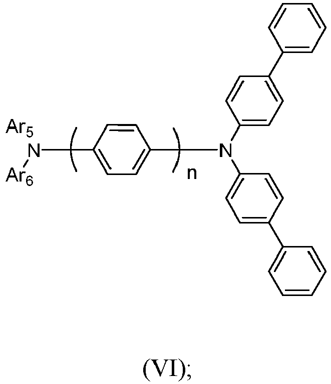
wherein n is 1, 2 or 3, and Ar₅ and Ar₆ are each independently selected from any one of substituted or unsubstituted C6 - C30 aryl; and
the doped material has a structure represented by Formula (VII): 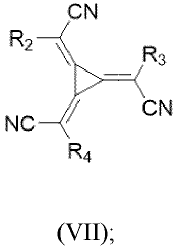 wherein R₂ to R₄ are each independently selected from any one of the following groups:

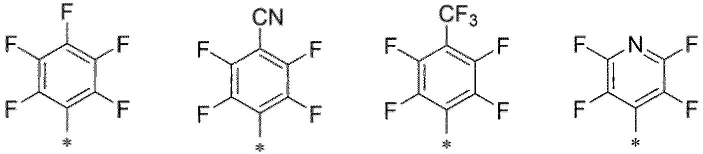

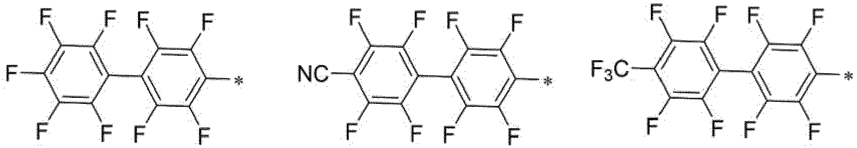

9. The organic electroluminescent device of claim 8, wherein the structure represented by Formula (VI) is selected from any one of the following structures:

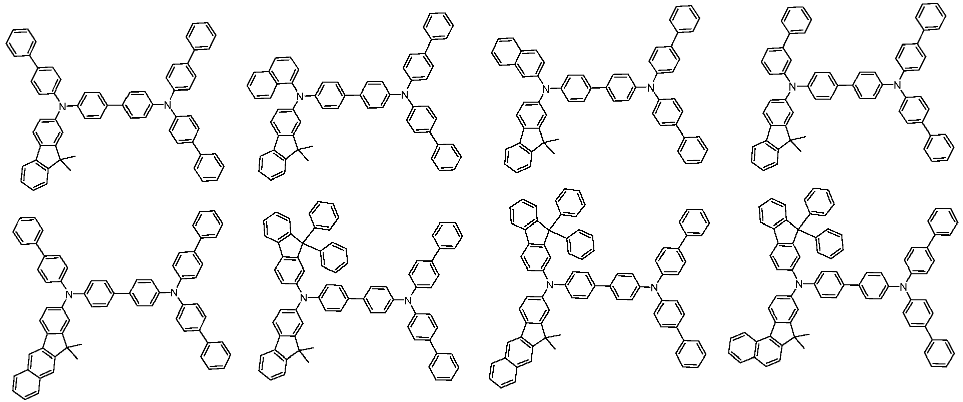

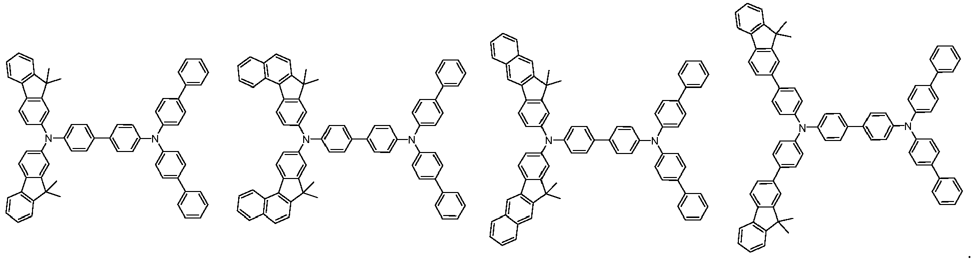

## Patentansprüche

1. Heterozyklische Verbindung mit einer Struktur entsprechend der Formel (I):
A-L-B Formel (I)
wobei L ausgewählt ist aus einer der folgenden Gruppen: 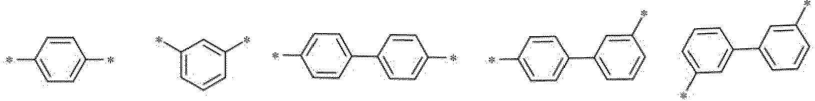 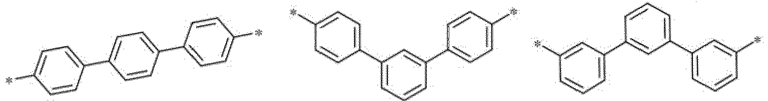 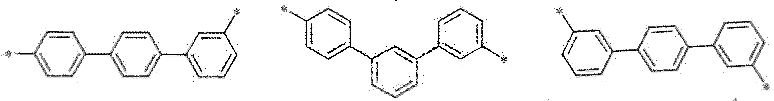 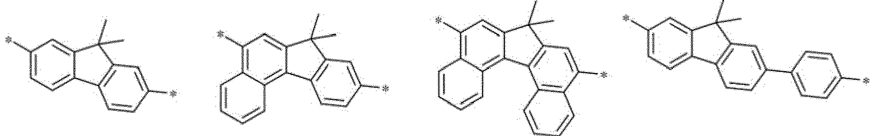 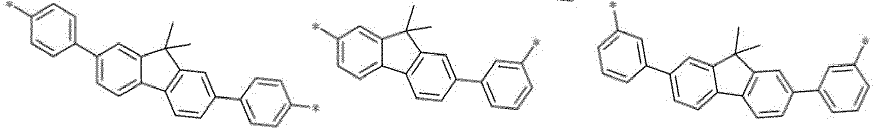 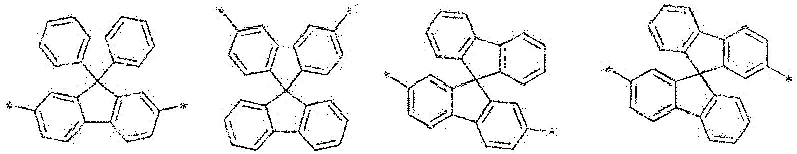 und
A und B jeweils unabhängig voneinander ausgewählt sind aus einer der Formeln (II) oder (III): 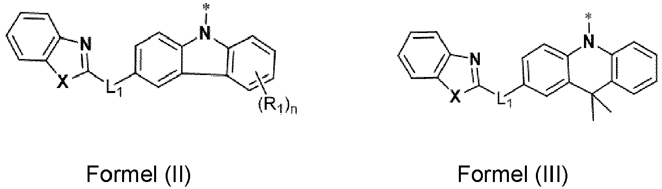
wobei X O oder S ist;
L₁ eine Einfachbindung, Phenyl oder Biphenyl ist;
n 0, 1, 2, 3 oder 4 ist; und
R₁ H, C₁-C₁₅-Alkyl oder C₆-C₃₀-Aryl ist, oder zwei benachbarte R₁-Gruppen unter Bildung einer Ringstruktur verbunden sind.

2. Heterozyklische Verbindung nach Anspruch 1, wobei A und B unabhängig voneinander ausgewählt sind aus einer der folgenden Formeln:

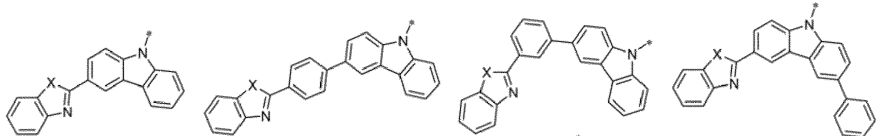

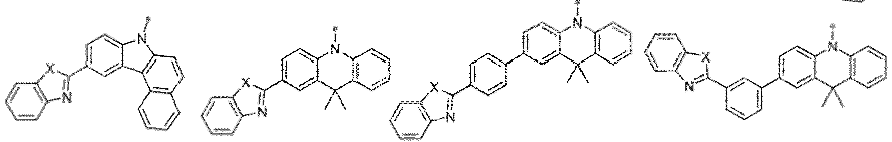

3. Heterozyklische Verbindung nach Anspruch 1, wobei die heterozyklische Verbindung ausgewählt ist aus einer der folgenden Verbindungen:

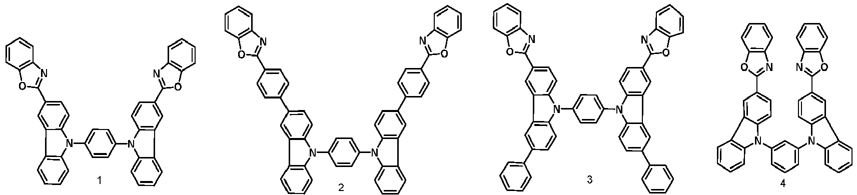

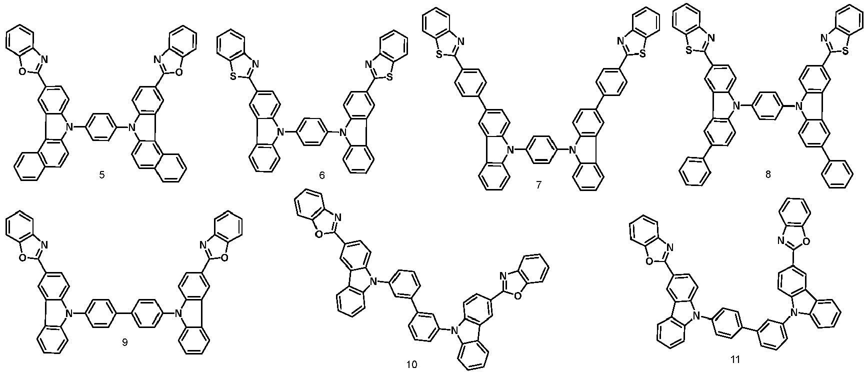

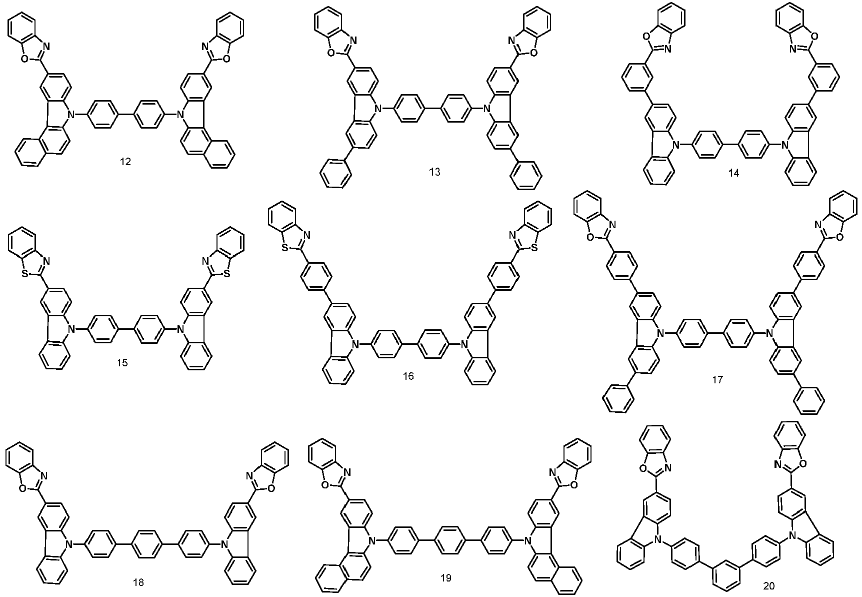

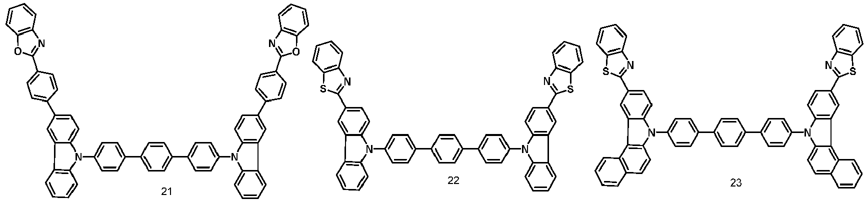

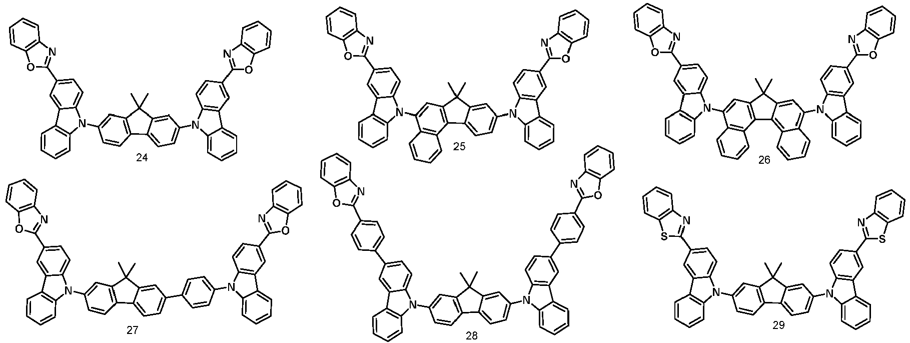

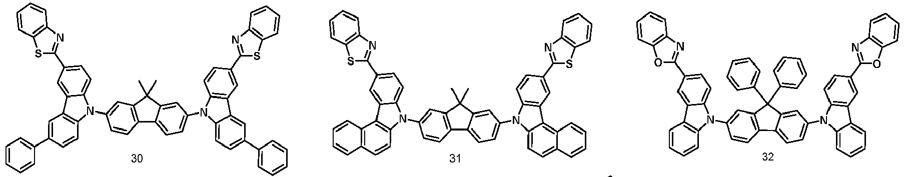

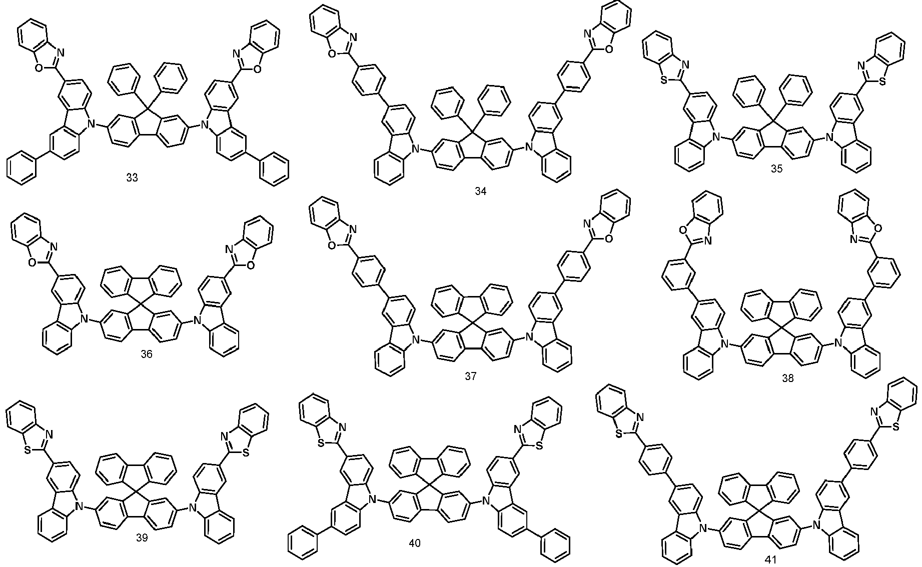

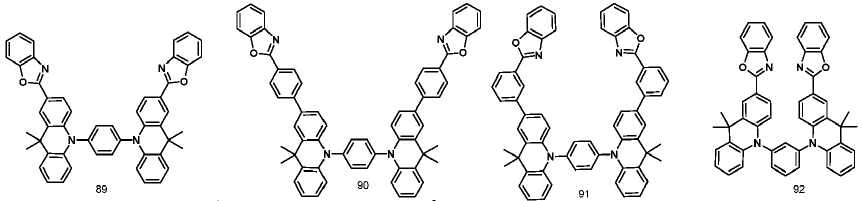

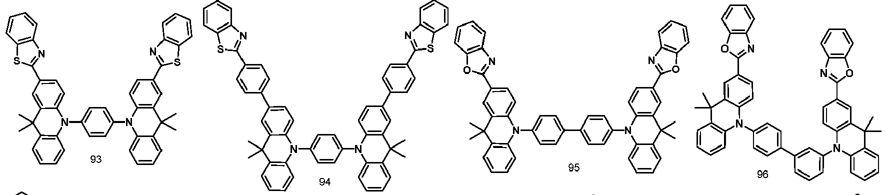

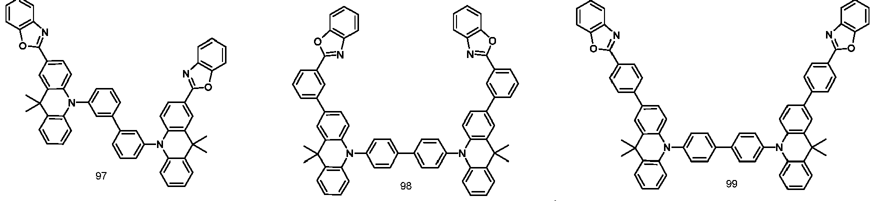

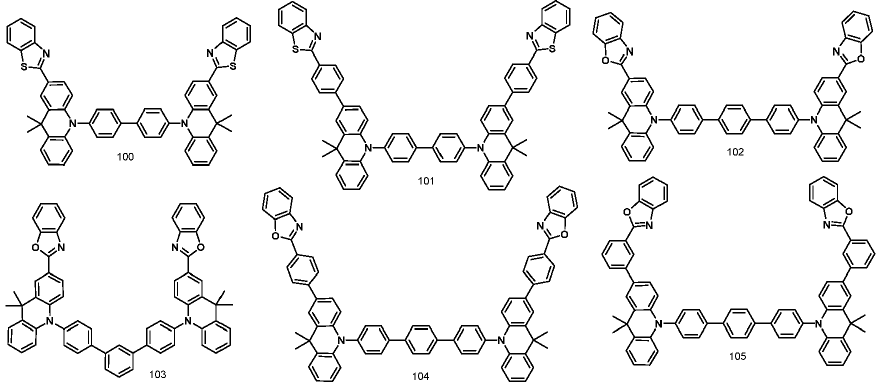

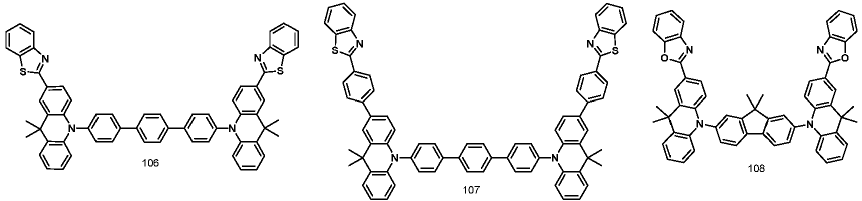

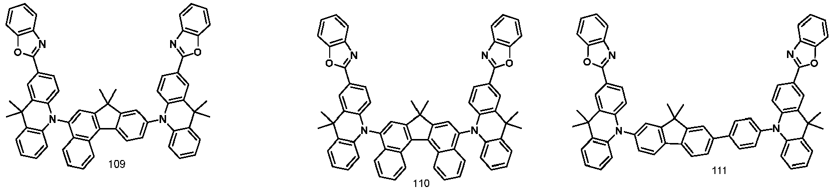

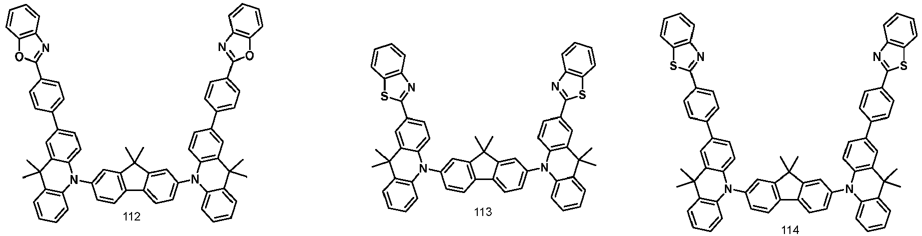

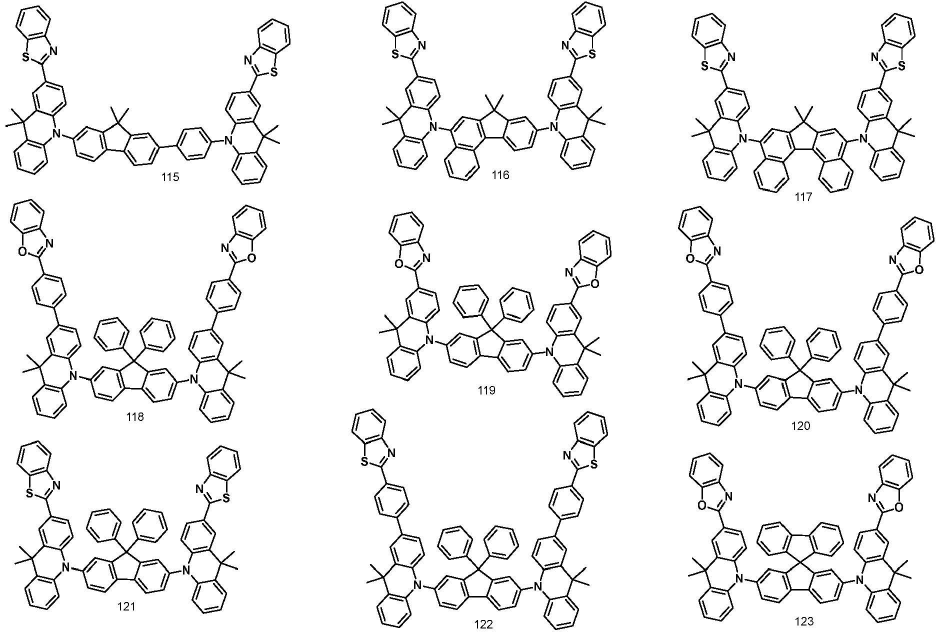

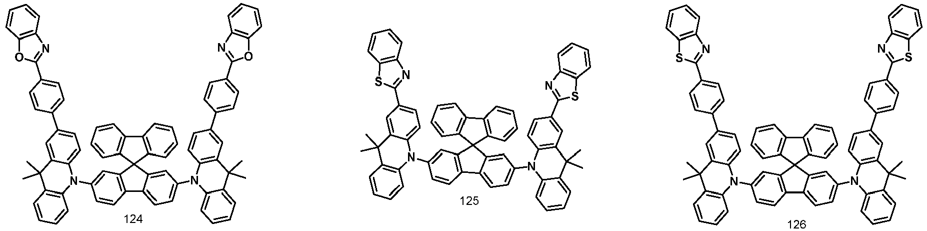

4. Organische elektrolumineszierende Vorrichtung, umfassend die heterozyklische Verbindung nach einem der Ansprüche 1 bis 3.

5. Organische elektrolumineszierende Vorrichtung nach Anspruch 4, wobei die organische elektrolumineszierende Vorrichtung eine Deckschicht, eine Kathode, eine organische Schicht, eine Anode und ein Substrat umfasst, wobei die Deckschicht die heterozyklische Verbindung nach einem der Ansprüche 1 bis 3 enthält.

6. Organische elektrolumineszierende Vorrichtung nach Anspruch 5, wobei die Deckschicht eine erste Deckschicht und eine zweite Deckschicht umfasst, wobei die erste Deckschicht die heterozyklische Verbindung nach einem der Ansprüche 1 bis 3 enthält, und die zweite Deckschicht eine durch die Formel (V) dargestellte Verbindung enthält: 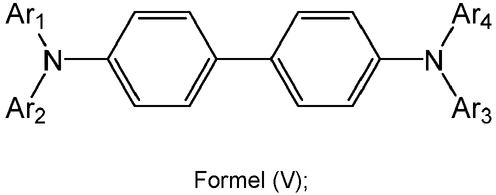 wobei Ar₁ bis Ar₄ jeweils unabhängig voneinander ausgewählt sind aus einem substituierten oder unsubstituierten C₆-C₃₀-Aryl.

7. Organische elektrolumineszierende Vorrichtung nach Anspruch 6, wobei die durch die Formel (V) dargestellte Verbindung ausgewählt ist aus einer der folgenden Strukturen:

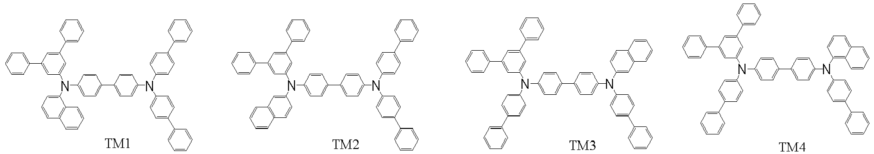

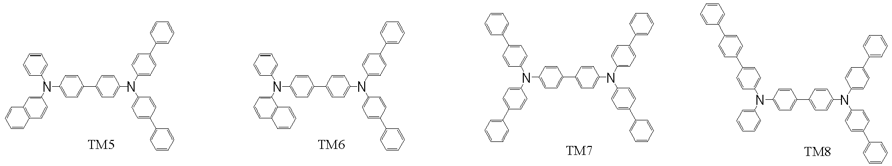

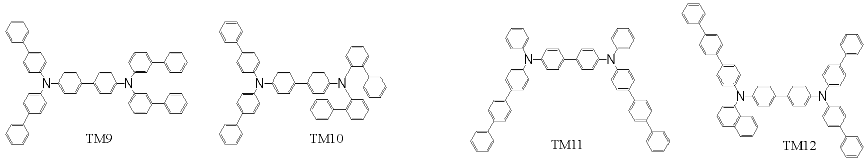

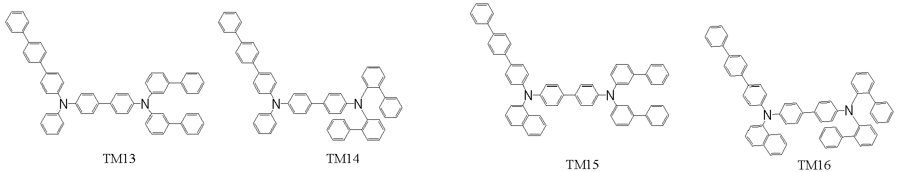

8. Organische elektrolumineszierende Vorrichtung nach Anspruch 5, wobei die organische Schicht eine Lochinjektionsschicht umfasst, umfassend ein Grundmaterial und ein dotiertes Material, wobei das Grundmaterial eine durch die Formel (VI) dargestellte Struktur aufweist: 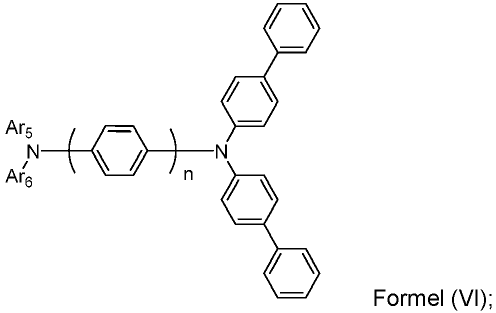
wobei n 1, 2 oder 3 ist, und Ar₅ und Ar₆ jeweils unabhängig voneinander ausgewählt sind aus einem substituierten oder unsubstituierten C₆-C₃₀-Aryl; und
das dotierte Material eine durch die Formel (VII) dargestellte Struktur aufweist: 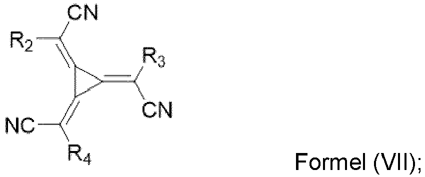 wobei R₂ bis R₄ jeweils unabhängig voneinander ausgewählt sind aus einer der folgenden Gruppen:

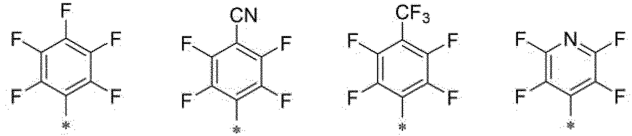

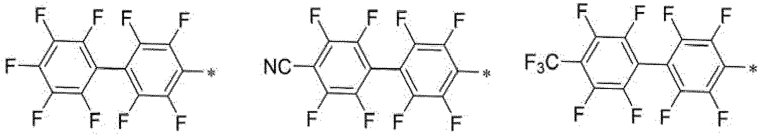

9. Organische elektrolumineszierende Vorrichtung nach Anspruch 8, wobei die durch die Formel (VI) dargestellte Struktur ausgewählt ist aus einer der folgenden Strukturen:

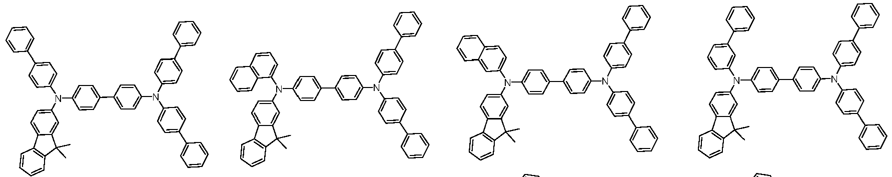

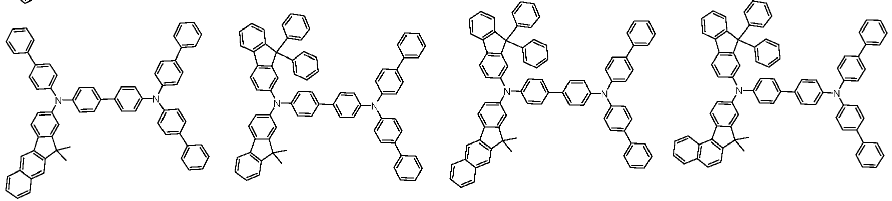

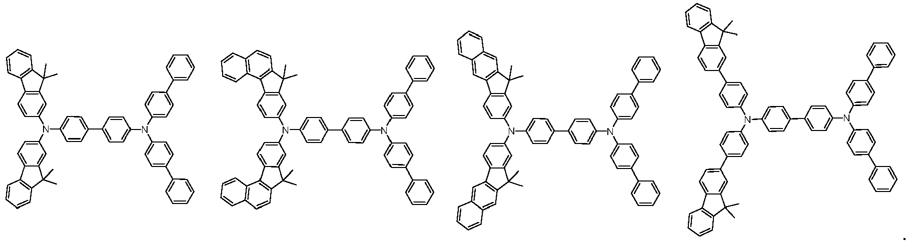

## Revendications

1. Composé hétérocyclique ayant une structure représentée par la Formule (I) :
A-L-B Formule (I)
dans laquelle L est sélectionné parmi l'un quelconque des groupes suivants : 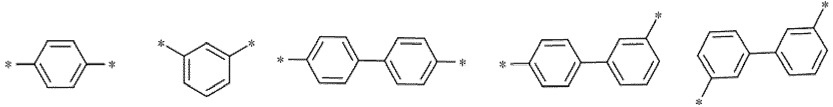 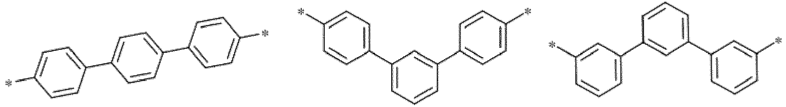 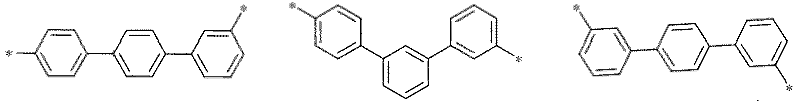 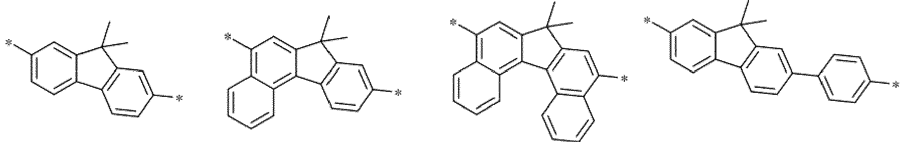 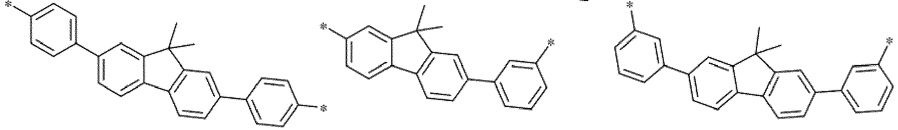 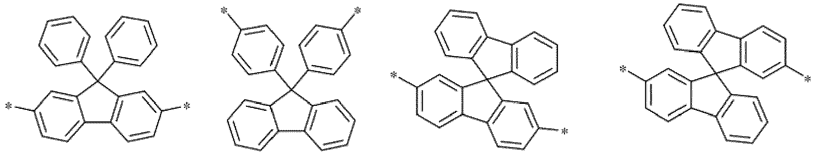 et
A et B sont chacun indépendamment sélectionnés parmi l'une quelconque de la Formule (II) ou de la Formule (III) : 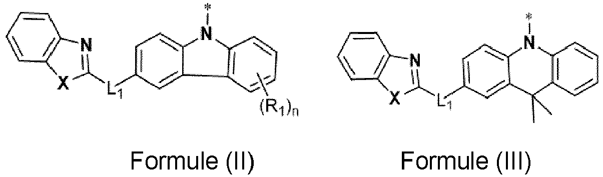
dans lesquelles X est O ou S ;
L₁ est une liaison simple, phényle ou biphényle ;
n vaut 0, 1, 2, 3 ou 4 ; et
R₁ est H, alkyle en C1 à C15 ou aryle en C6 à C30, ou deux groupes R₁ adjacents sont liés pour former une structure cyclique.

2. Composé hétérocyclique selon la revendication 1, dans lequel A et B sont indépendamment sélectionnés parmi l'une quelconque des formules suivantes :

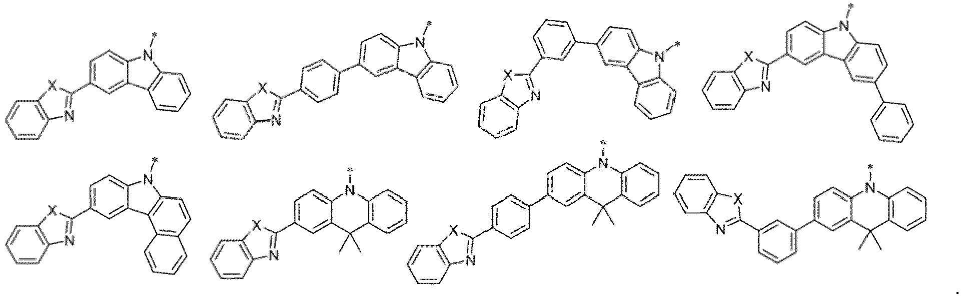

3. Composé hétérocyclique selon la revendication 1, dans lequel le composé hétérocyclique est sélectionné parmi l'un quelconque des composés suivants :

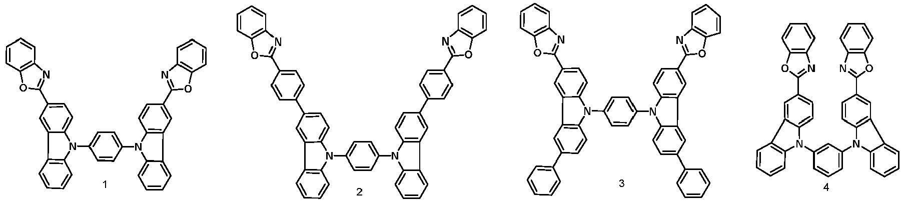

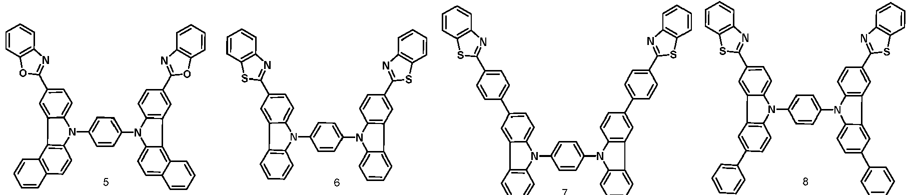

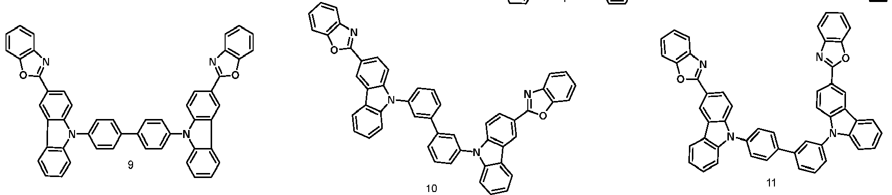

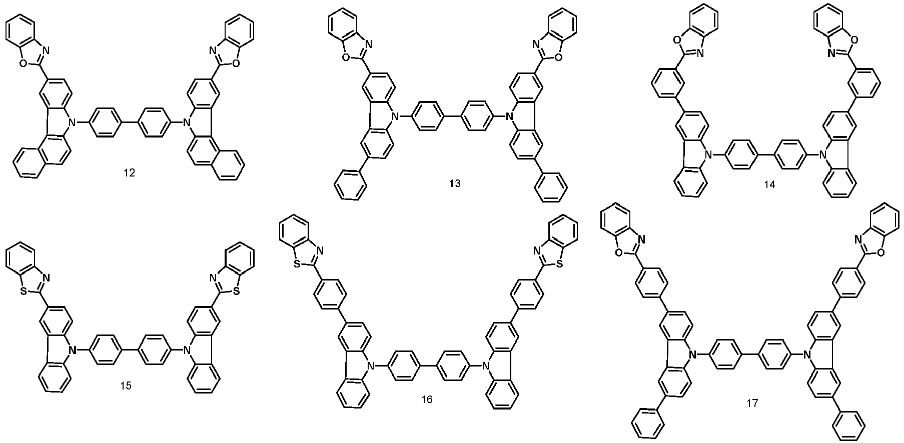

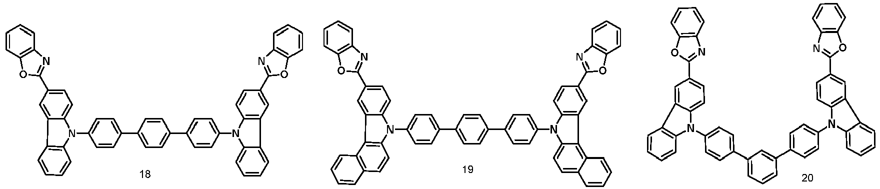

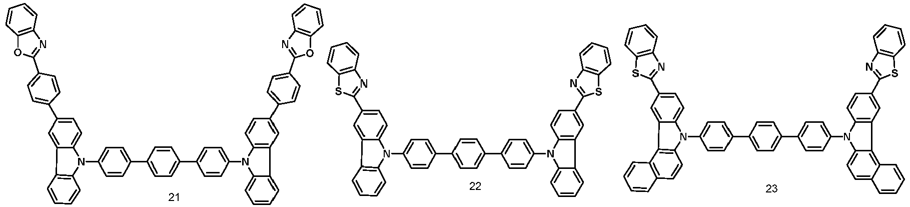

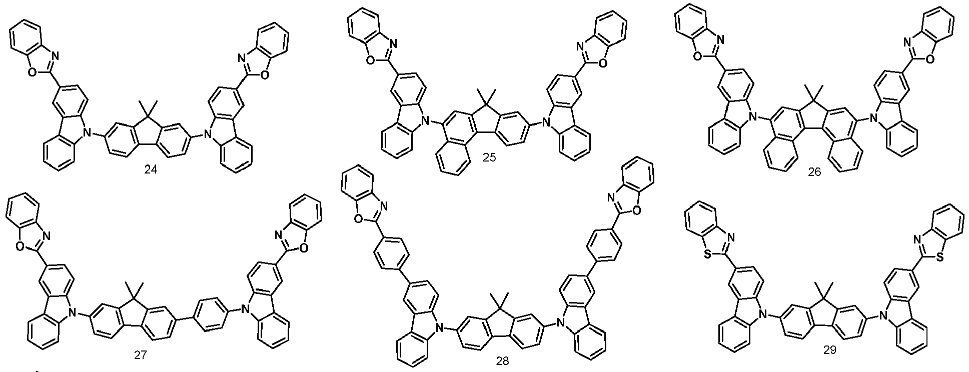

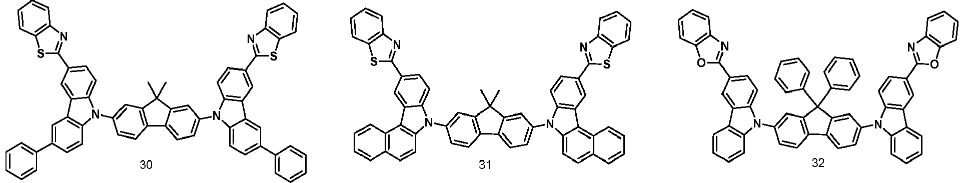

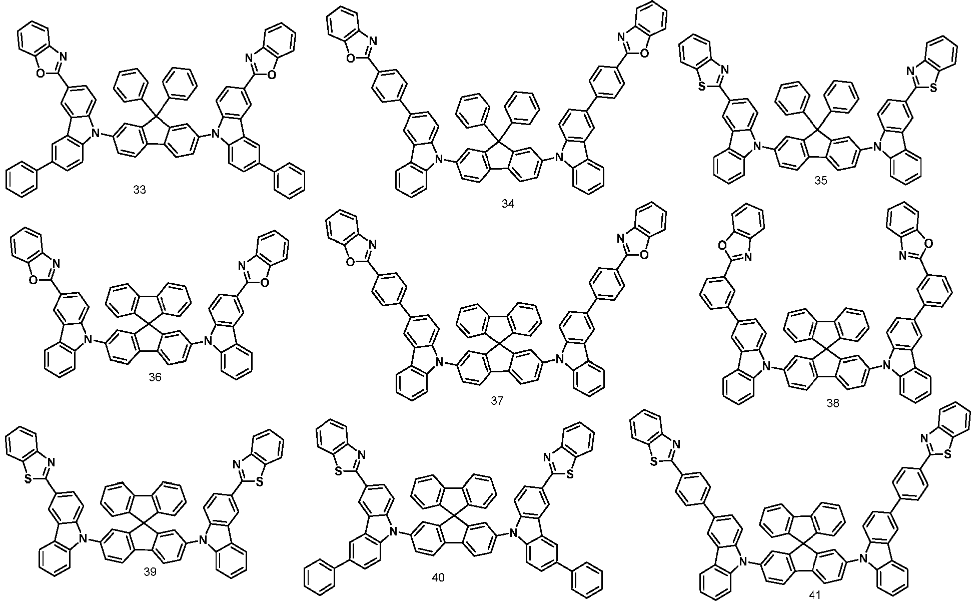

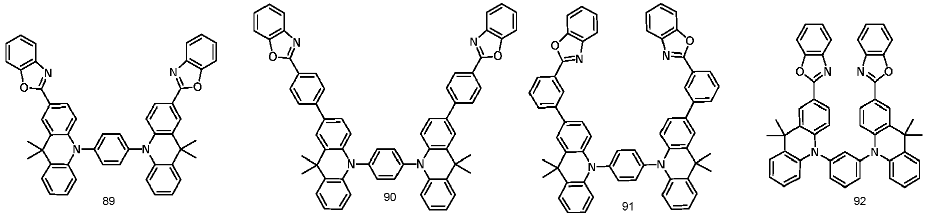

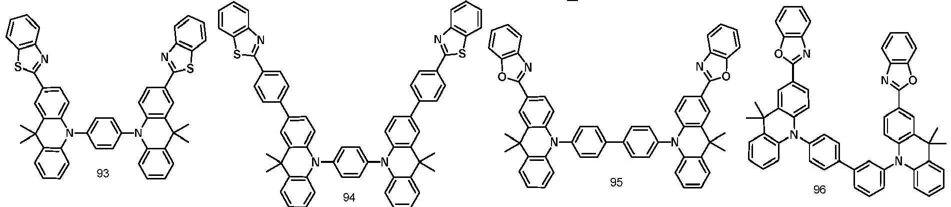

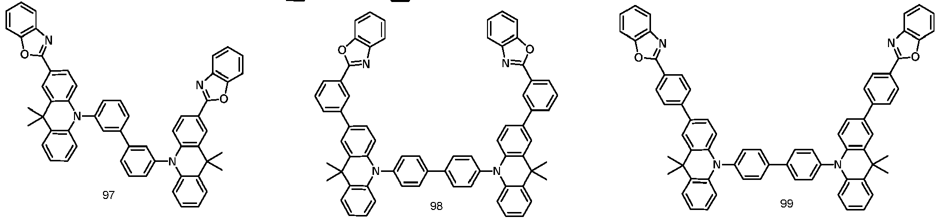

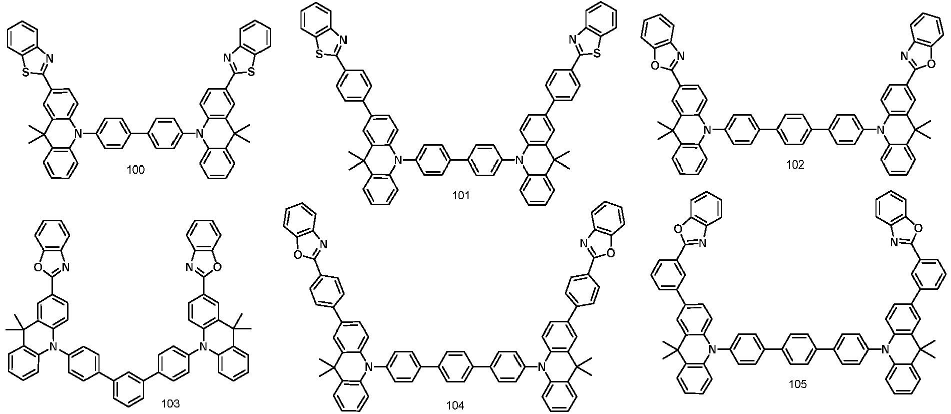

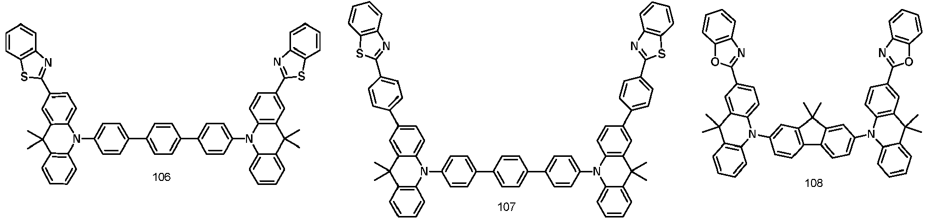

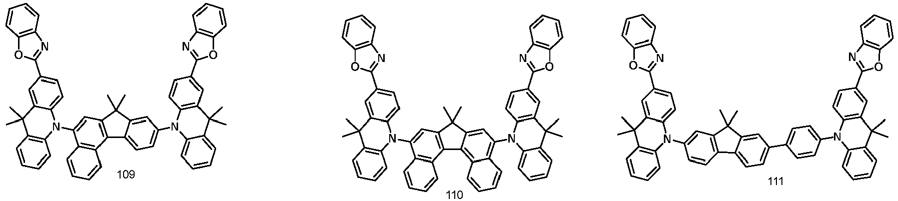

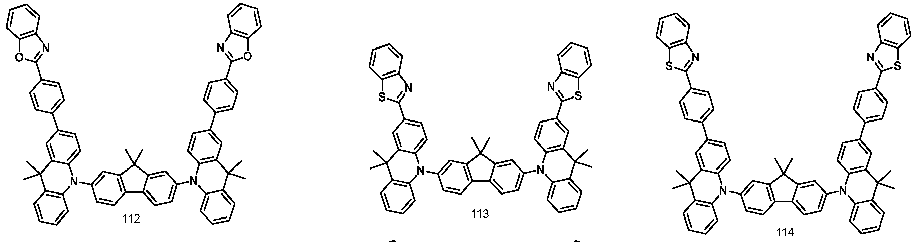

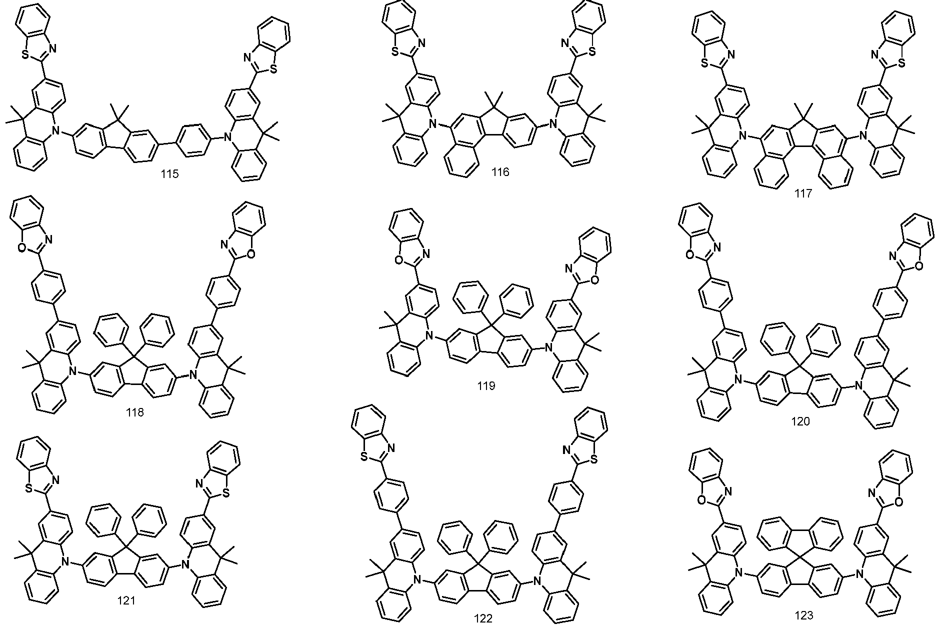

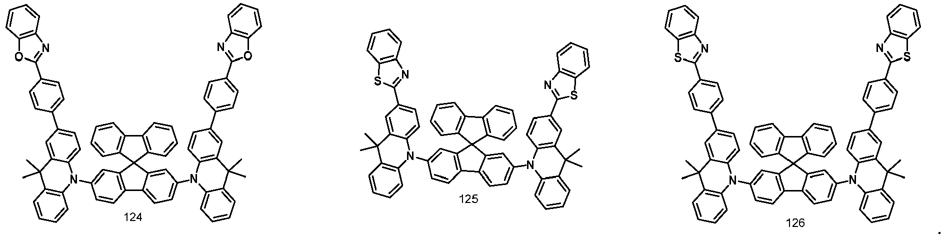

4. Dispositif électroluminescent organique, comprenant le composé hétérocyclique selon l'une quelconque des revendications 1 à 3.

5. Dispositif électroluminescent organique selon la revendication 4, dans lequel le dispositif électroluminescent organique comprend une couche de recouvrement, une cathode, une couche organique, une anode et un substrat, dans lequel la couche de recouvrement contient le composé hétérocyclique selon l'une quelconque des revendications 1 à 3.

6. Dispositif électroluminescent organique selon la revendication 5, dans lequel la couche de recouvrement comprend une première couche de recouvrement et une seconde couche de recouvrement, dans lequel la première couche de recouvrement contient le composé hétérocyclique selon l'une quelconque des revendications 1 à 3, et la seconde couche de recouvrement contient un composé représenté par la Formule (V) : 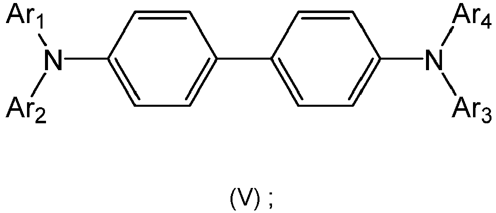 dans laquelle Ar₁ à Ar₄ sont chacun indépendamment sélectionnés parmi l'un quelconque d'un aryle en C6 à C30 substitué ou non substitué.

7. Dispositif électroluminescent organique selon la revendication 6, dans lequel le composé représenté par la Formule (V) est sélectionné parmi l'une quelconque des structures suivantes :

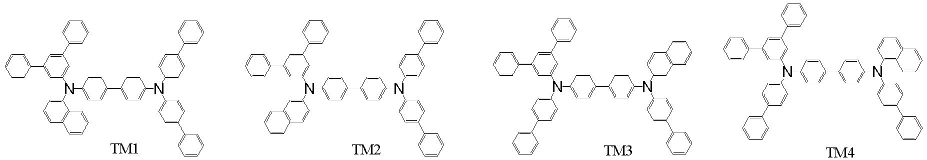

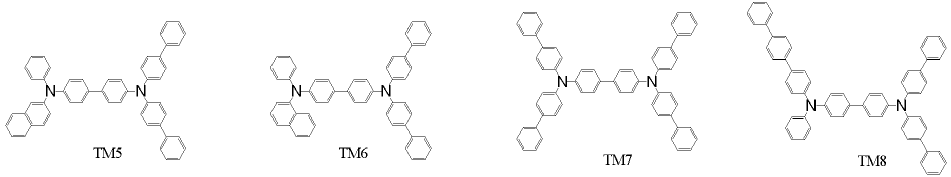

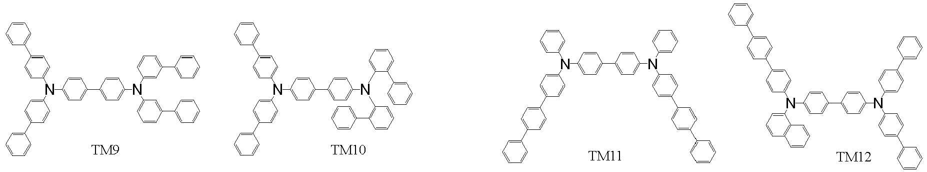

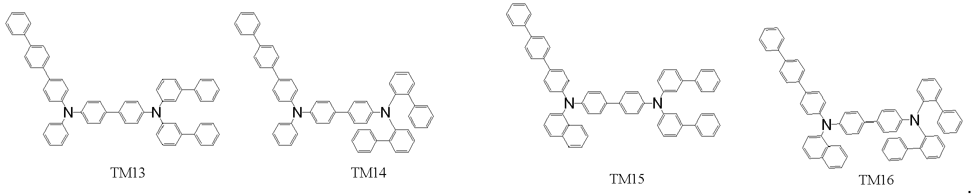

8. Dispositif électroluminescent organique selon la revendication 5, dans lequel la couche organique comprend une couche d'injection de trous comprenant un matériau hôte et un matériau dopé, dans lequel le matériau hôte possède une structure représentée par la Formule (VI) : 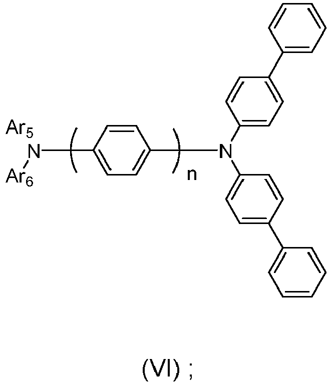
dans laquelle n vaut 1, 2 ou 3, et Ar₅ et Ar₆ sont chacun indépendamment sélectionnés parmi l'un quelconque d'un aryle en C6 à C30 substitué ou non substitué ; et
le matériau dopé possède une structure représentée par la Formule (VII) : 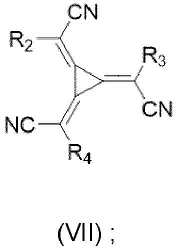 dans laquelle R₂ à R₄ sont chacun indépendamment sélectionnés parmi l'un quelconque des groupes suivants :

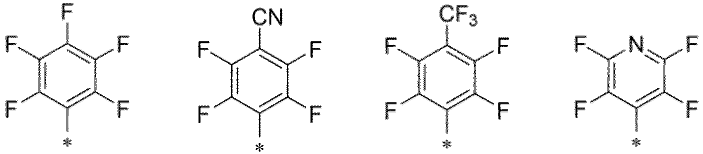

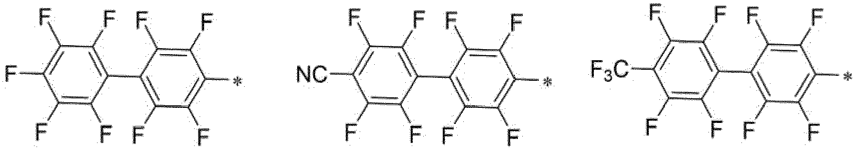

9. Dispositif électroluminescent organique selon la revendication 8, dans lequel la structure représentée par la Formule (VI) est sélectionnée parmi l'une quelconque des structures suivantes :
